# EUROPEAN PATENT APPLICATION

(11) **EP 0 712 847 A1**
(43) Date of publication of application: **22.05.1996**
(21) Application number: 94922352.3
(22) Date of filing: 10.08.1994
(51) Int. Cl.: C07D 233/64, C07D 233/88, C07D 233/68, C07D 233/90, C07D 233/92, C07D 233/93, C07D 233/94, C07D 401/10, C07D 401/12, A01N 43/50

(54) **IMIDAZOLE DERIVATIVE, PROCESS FOR PRODUCING THE SAME, AND PEST CONTROL DRUG**

(30) Priority: 11.08.1993 JP 219057/93; 25.10.1993 JP 288687/93
(71) Applicant: NIPPON SODA CO., LTD., Chiyoda-ku, Tokyo 100 (JP)
(72) Inventor: HAGIWARA, Kenji Odawara Research Center, Odawara-shi Kanagawa 250-02 (JP); TAKADA, Mitsumasa Odawara Research Center, Odawara-shi Kanagawa 250-02 (JP); MARUYAMA, Michiaki Odawara Research Center, Odawara-shi Kanagawa 250-02 (JP); MATSUDA, Michihiko Odawara Research Center, Odawara-shi Kanagawa 250-02 (JP); HATANO, Renpei Odawara Research Center, Odawara-shi Kanagawa 250-02 (JP); MITSUI, Jun Haibara Agricultura Research Lab.,, Haibara-gun Shizuoka 421-04 (JP); SANO, Shinsuke Odawara Research Center, Takada, Odawara-shi Kanagawa 250-02 (JP)
(74) Representative: Wibbelmann, Jobst, Dr., Dipl.-Chem.
(86) International application number: JP9401319
(87) International publication number: WO9504724

(57) **Abstract**

An imidazole derivative represented by general formula [I], which has insecticidal, acaricidal and agrohorticultural bactericidal activities, wherein R¹ represents phenyl which may be substituted by halogen, C₁-C₆ alkyl, C₁-C₆ haloalkyl, C₁-C₆ alkoxy, C₁-C₆ haloalkoxy, C₁-C₆-alkoxy-substituted C₁-C₆ alkoxy, optionally substituted phenyl, optionally substituted pyridyl, optionally substituted phenoxy, optionally substituted pyridyloxy, C₂-C₆ alkenyloxy, C₂-C₆ haloalkenyloxy, C₂-C₆ alkynyloxy, C₂-C₆ haloalkylnyloxy, nitro, C₁-C₆ alkylthio, C₁-C₆ alkylsulfinyl, C₁-C₆ alkylsulfonyl, di(C₁-C₆ alkyl) sulfamoyl, cyano, C₁-C₆ alkoxycarbonyl or C₁-C₆ alkylcarbonyl, or which may be fused with a benzene ring to form naphthyl; R² represents C₁-C₆ alkoxycarbonyl, C₁-C₆-alkoxy-substituted C₁-C₆ alkoxycarbonyl, cyano r¹r²NCO (wherein r¹ and r² represent each hydrogen, C₁-C₆ alkyl or optionally substituted phenyl), C₁-C₆ alkylthiocarbonyl, C₁-C₆ alkoxycarboimidoyl, carboxy, halogen, C₁-C₆ haloalkyl, formyl or nitro; R³ represents hydrogen, optionally halogenated phenyl, C₁-C₆ haloalkyl, C₁-C₁₂ alkyl, benzoyl, C₁-C₆ alkoxycarbonyl, C₁-C₆ alkylcarbonyl, aralkyl or C₃-C₇ cycloalkyl; and R⁴ represents hydrogen, C₁-C₆ alkyl (which may be substituted by C₁-C₆ alkoxy, optionally substituted aralkyloxy, C₁-C₆-alkoxy-substituted C₁-C₆ alkoxy, C₁-C₆ acyloxy, C₁-C₆ alkoxycarbonyloxy, C₁-C₆ acylamido, C₂-C₆ alkenyloxy, C₂-C₆ alkynyloxy, r³SOₙ (wherein r³ represents C₁-C₆ alkyl or optionally substituted phenyl, and n represents an integer of 0 to 2), optionally substituted benzoyl, C₁-C₆ alkoxycarbonyl, di(C₁-C₆ alkyl) phosphinyl, cyano or thiocyanato), aralkyl, C₂-C₆ alkenyl, C₂-C₆ alkynyl, C₁-C₆ alkylcarbonyl or cyano, provided R² and R⁴ may form together a ring.

## Description

### Technical Field:

The present invention relates to new derivatives of imidazole, the process for producing said compounds and the plants protecting agents against injurious living things.

### Background Art:

Since in cultivations of agricultural products and horticultural products a great number of agrochemicals and agents are being used to protect and to exclude the diseases and the damage caused by the injurious living things such as bacilli and insects, the efficacy of the chemicals and agents are still not sufficient and further for the progression of the new disease bacilli and the new harmful insects, those resistivity to said agents have evolved, the application of said agrochemicals and agents become more limited in the narrow range.

In another aspect, because said chemicals and said agents cause frequently a drug injury and a contamination to the plant and also the toxicity of said chemicals and said agents show unpreferable affects on human beings, animals, fishes and shells, we have some time encountered with the defective chemicals and agents for such a protective use for plants harvest, and therefore, it is a strong request to have a agrochemical and a agent which is used in safety and exhibits less defects, in other word, with minimal disruption of the environment.

A similar compound to the compound of this invention has been published in DE 3,902,772 and was described that 2-halo-4-phenylimidazol-5-carboxylic acid derivatives demonstrate an antidote effect for the herbicides, however, no description about the activity for a insecticide, a acaricide and a fungicide was cited in its.

In this connection, the following reaction was described in Liebigs. Ann. Chem.(1975) 160-194.

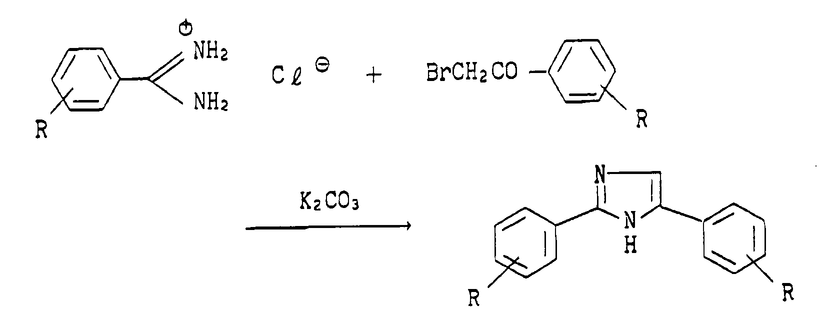

### Disclosure of the Invention:

The object of this invention is to provide new compounds which may be synthesized reasonably by industries and have assured efficacy for the protection and the exclusion of injurious living things.

This invention provides the protecting agents of plants against injurious living things, the process for producing the compounds and the compounds of imidazole derivatives comprising of the structure of the Formula I : wherein 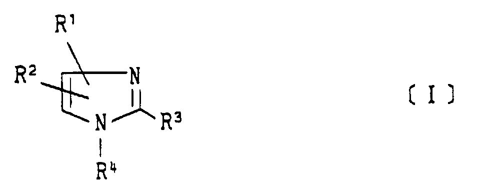
R¹ represents phenyl which group is optionally substituted with one or more substituents selected from halogen, C₁₋₆ alkyl, C₁₋₆ haloalkyl, C₁₋₆ alkoxy, C₁₋₆ haloalkoxy, C₁₋₆ alkoxy-substituted alkoxy, optionally substituted phenyl, optionally substituted pyridyl, optionally substituted phenoxy, optionally substituted pyridyloxy, C₂₋₆ alkenyloxy, C₂₋₆ haloalkenyloxy, C₂₋₆ alkynyloxy, C₂₋₆ haloalkynyloxy, nitro, C₁₋₆ alkylthio, C₁₋₆ alkylsulfinyl, C₁₋₆ alkylsulfonyl, C₁₋₆ alkylsulfamoyl, cyano, C₁₋₆ alkoxycarbonyl or C₁₋₆ alkylcarbonyl, or said R¹ represented by substituted phenyl optionally forming naphthyl condensed with benzene ring ; and
R² represents C₁₋₆ alkoxycarbonyl, C₁₋₆ alkoxy C₁₋₆ alkoxycarbonyl₁ cyano, r¹ r² NCO- which (r¹ and r² severally and independently is hydrogen atom, C₁₋₆ alkyl or optionally substituted phenyl,) C₁₋₆ alkylthiocarbonyl, C₁₋₆ alkoxycarboimidoyl, carboxy, halogen, C₁₋₆ haloalkyl, formyl or nitro ; and
R³ represents phenyl optionally substituted with halogen atoms, C₁ ₋₆ haloalkyl. C₁₋₁₂ alkyl, benzoyl, C₁₋₆ alkoxycarbonyl, C₁₋₆ alkylcarbonyl, aralkyl, C₃₋₆ cycloalkyl or hydrogen atom ; and
R⁴ represents hydrogen atom or C₁₋₆ alkyl which alkyl is optionally substituted with substituents selected from optionally substiteted aralkyoxy, C₁₋₆ alkoxy, C₁₋₆ alkoxy C₁₋₆ alkoxy, C₁₋₆ acyloxy, C₁₋₆ alkoxycarbonyloxy, C₁₋₆ acylamido, C₂₋₆ alkenyloxy, C₂₋₆ alkynyloxy, r³ SOn- (having r³ of C₁₋₆ alkyl or optionally substituted phenyl and having an integer of from 0 to 2), optionally substituted benzoyl, C₁₋₆ alkoxycarbonyl, di-C₁₋₆ alkylphosphinyl, cyano or thiocyanate, and further represents aralkyl, C₂₋₆ alkynyl, C₂₋₆ alkenyl, C₁₋₆ alkylcarbonyl, or cyano, and optionally said group combined with R ² to form a ring-structure.

For example, substituents which substitutes phenyl in R¹ comprising phenyl, pyridyl, phenoxy, and pyridyloxy are illustrated such as halogen, C₁₋₆ haloalkyl and the like.

Substituents which substitutes alkyl in R⁴ comprising aralkyloxy, benzoyl and phenyl in r³ are halogen and the like as an example.

The processes for producing the compounds of the present invention are demonstrated as follows.
(a) 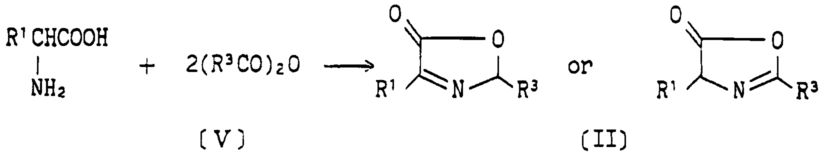 (wherein R¹ and R³ represent the same definition as mentioned above)
   The reactions are carried out on heating in an inert solvent such as acetonitrile, esters represented with a ethyl acetate and aromatic hydrocarbons represented with toluene and also in the absence of solvent. After the reaction, reaction mixture may be isolated otherwise this crude reaction mixture is used for the next step. 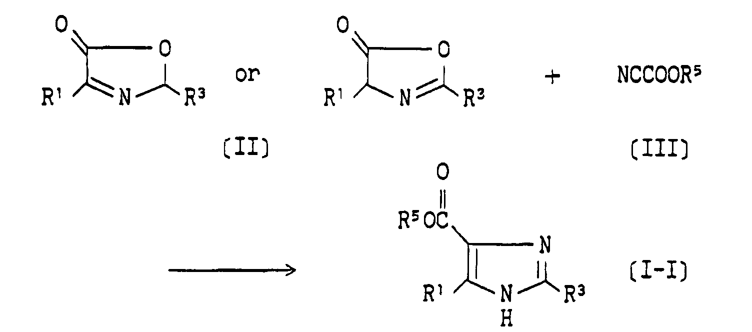
(b) 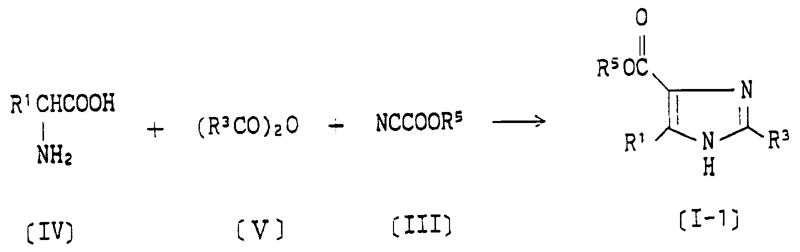 (wherein R¹, R³ and R⁵ represent the same definition described above)
   The reaction is proceeded in an inert solvent under existence of a trivalent phosphorus compounds as a catalyst and a dehydrating agent at the temperature of from room temperature to 120°C. The quality and the quantity of trivalent phosphorus compounds and the inert solvent are same as described above. Dehydrating agents are acid anhydrides and a dicyclohexylcarbodiimide and the like, and the more preferable agent is the compounds illustrated in the formula (V). 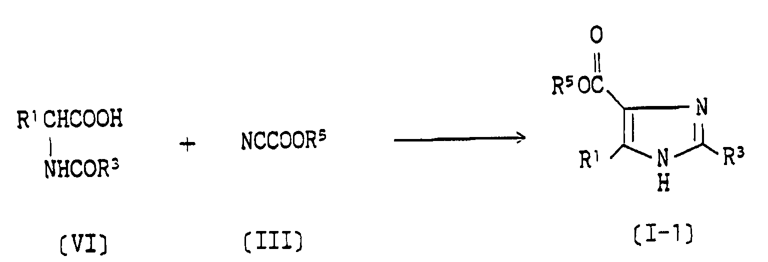 (wherein R¹, R³ and R⁵ represent the same as described above)
   The reaction is proceeded in the' inert solvent in the presence of a trivalent phosphorous catalyst and a dehydrating agent at room temperature to 120 °C, and the similar quality, quantity of phosphite and of solvent are used to above preparation and as a dehydrating agent may be acid anhydrides, dicyclohexylcarbodiimide and the like.
(c) The conversion from the compounds which R³ is alkoxycarbonyl in Formula I. 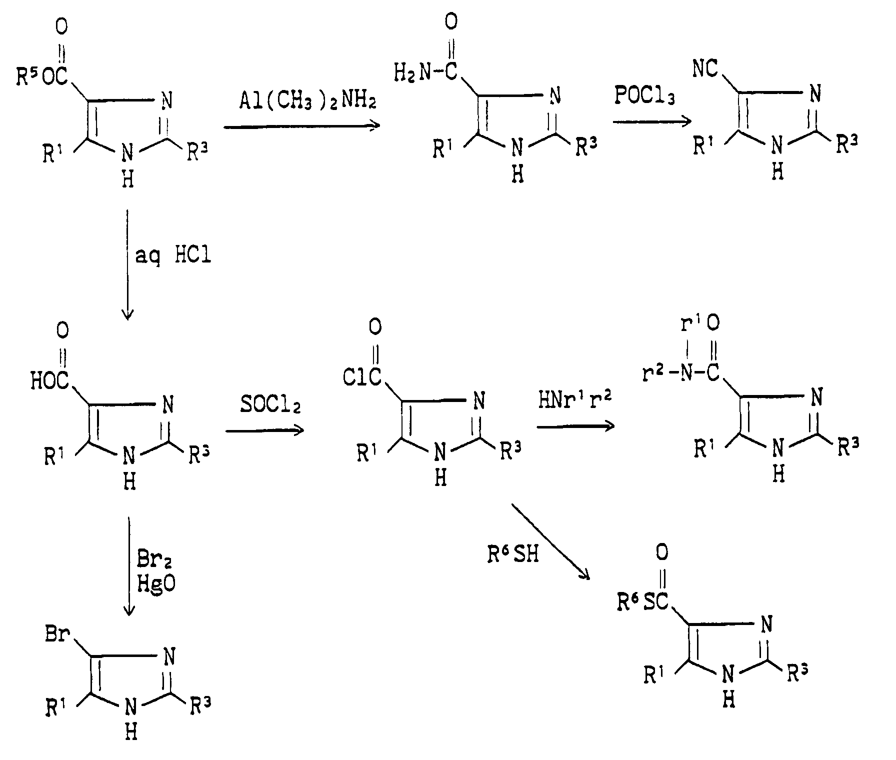 (wherein R¹, R³, R⁵ r¹ and r² represent the same definition above described and R⁶ represents C₁₋₆ alkyl)
(d) In addition, the compounds of this invention is produced by the following method. 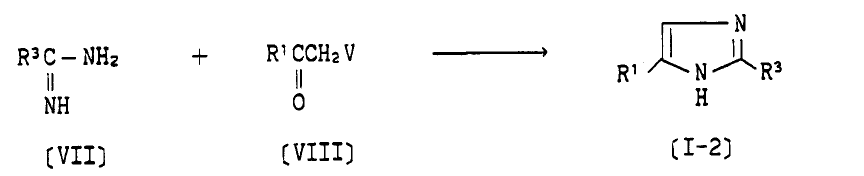 (wherein R¹ and R³ represent the same as above and V represents a halogen atom)
   The reaction is proceeded in the presence of bases with or without solvent at the temperature in the range of -50 to 150 °C.
   As a base herein used is selected from the group of alkali-metal hydroxides, alkali-metal carbonate, sodium hydride, tertiary amines, pyridine and the like. In an alternative method, amidines is employed as a raw-material and illustrated in formula [VI] herein and can act itself as a base when they are used more than two moles equivalent. Especially in the case of R³ representing haloalkyl, high yield is obtainable when the amidines having the formula [VI] is applied more than two molar equivalent as an alternative of a base. It is preferable to use acetone, dimethylformamide, acetonitrile, ethyl acetate as a solvent.
(e) The conversion from the compounds displayed in the formula [I-2] 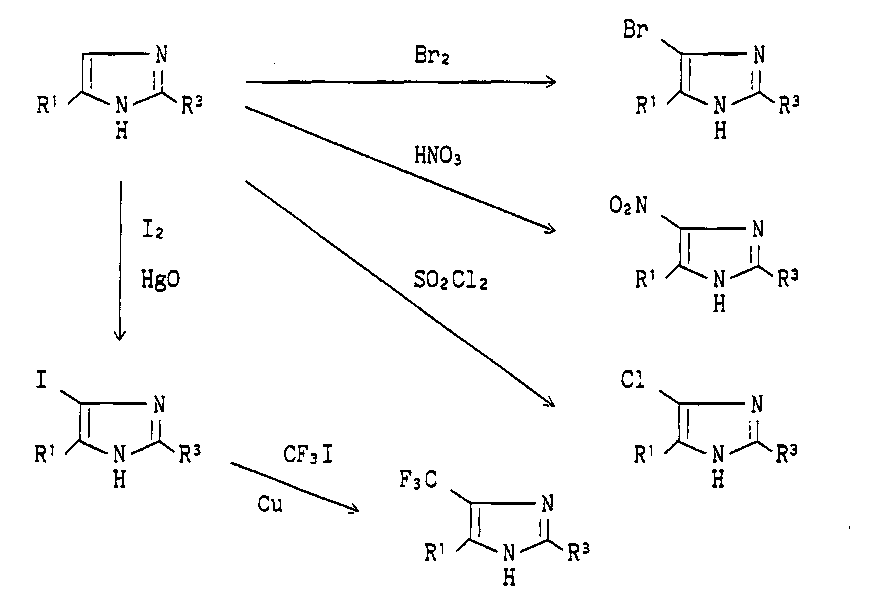 (wherein R¹ and R³ represents the same as described above)
(f) The conversion of NH-group in a imidazole ring 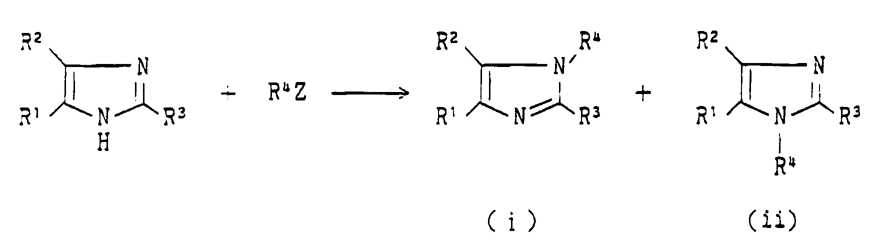 (wherein R¹, R², R³ and R⁴ represent the same as above, and Z represents a withdrawal substituents) The substituents withdrawn indicated as follows, halogen atoms such as chlorine and bromine, OSO₂r⁴(r⁴ is alkyl such as methyl and the like and optionally substituted phenyl such as p-tolyl and the like) etc.
   The reaction is proceeded in an inert solvent and the presence of a base at temperature from -50 to 200°C, and usually two type (i) and (ii) of the products are obtained respectively.

The bases used in this reaction are listed as follows, a metal alkoxide such as potassium t-butoxide,a alkali metal hydroxide such as sodium hydroxide, potassium carbonate, a alkali metal hydride such as sodium hydride and an organic base such as triethylamine and pyridine.

For example, solvents used in this reaction are also listed as follows, tetrahydrofuran, acetone, acetonitrile, ethyl acetate, chloroform, dimethylformamide, dimethylsulfoxide and ethyl alcohol etc.

In any case, it is possible to obtain the targeted compounds by usual post-treatments.

The chemical structure of the compounds in the present invention was determined by analyses of infrared absorption, nuclear magnetic resonance, mass spectrometry and the like.

### Best Mode of Carrying Out the Invention:

The following examples describe and illustrate the methods for the preparation of the compounds of the present invention, as well as other aspects of the present invention in more detail hereinafter.

### Example 1

### The synthesis of 4-ethoxycarbonyl-5-phenyl-2-trifluoromethyl imidazole (compoun No. II-45)

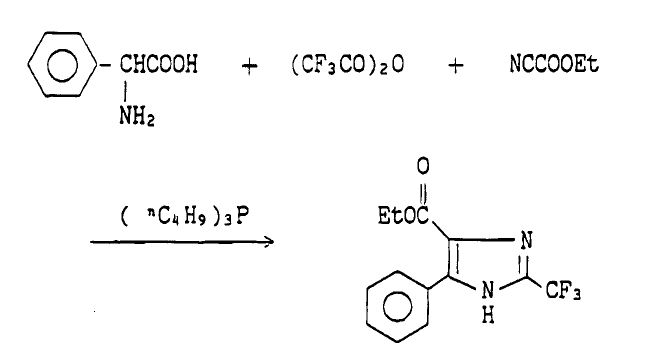

To a stirring at room temperature, 30.20g of phenylglycine (0.2 moles) was added dropwise 84.00g (0.4mol) of trifluoroacetic anhydride and the mixture was gradually heated. After refluxing for 2 hours, the mixture was concentrated under reduced pressure by a rotary-evaporator. 30ml of toluene was poured into the residue in the evaporator and again the solution was concentrated under reduced pressure. Crude 4-phenyl-2-trifluoromethyl-Δ³-oxazolidin-5-one was obtained.

Under stirring to a mixture of the crude oxazolidine, 200ml toluene and 19.80g ethyl cyanoformate (0.2 mol) was added dropwise 13.47g of tributyl phosphine (0.067 mol) and then the reaction mixture was heated gradually and was refluxed for 2 hours.

Then removed into an rotary-evaporator, the reaction mixture was concentrated under reduced pressure and the residue was purified by a silicagel columnchromatography with the solvent mixture which has the ratio benzene to ethyl acetate of 5 to 1 and finally 47.93g of the object compound was obtained. (yield 84%,) m.p. 148-152°C.

### Example 2

### The synthesis of 4-carbamoyl-5-phenyl-2-trifluoromethylimidazole (Compound No. 11-53) and the synthesis of 4-ethoxycarbimidoyl-5-phenyl-2-trifluoromethylimidazole (Compound No. II-52)

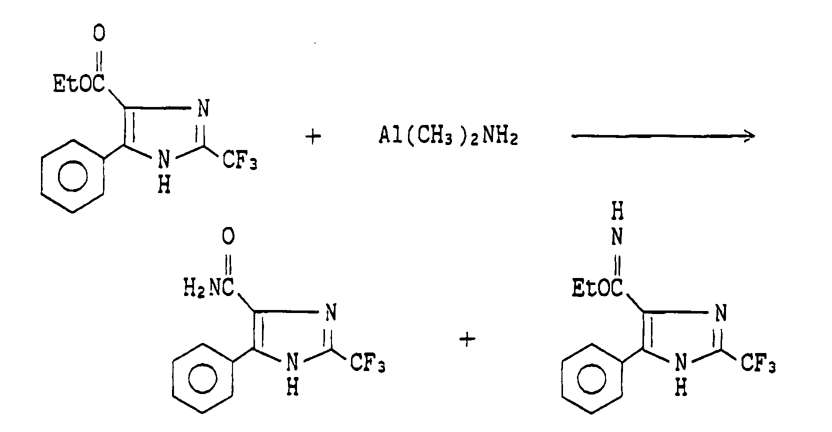

Trimethyl alminium (2.0M hexane solution, 38.3ml,0.076mol) was charged into a flask under atmosphere of nitrogen, and the separately prepared solution of 1.3g of ammonia (0.076 moles) disolved in dry tetrahydrofuran 100ml was poured dropwise into said hexane solution under a ice-cooling and the mixture was stirred at room temperature for one hour and then 12.41g of 4-ethoxycarbonyl-5-phenyl-2-trifluoromethyli midazole (0.0437 moles) dissolved in 180ml of tetrahydrofuran was added dropwise into said mixture. And the reaction mixture was stirred at room temperature for 2 hours and was refluxed for one hour. After cooling the solution, dilute aqueous hydrochloric acid was added dropwise to decompose organo aluminium compounds under ice-cooling. When the foaming has ceased 150ml of water was added into said mixture to separate thereof into two phases and the phase of tetrahydrofran was dried by the desiccant of anhydrous magnesium sulfate, filtered, concentrated under vacuum and the residue obtained was purified by silica gei column chromatography using a solvent of 20 to 1 chloroform-methanol and two species of compounds was obtained.
4-carbamoyl-5-phenyl-2-trifluoromethylimidazole (5.65g yield 51%) m.p. 115-118°C
4-ethoxycarbimidoyl-5-phenyl-2-trifluoromethylimidazole (0.85g yield 7%) m.p. 177- 178 °C

### Example 3

### The synthesis of 4-cyanophenyl-2-trifluoromethylimidazole (Compound No. II-54)

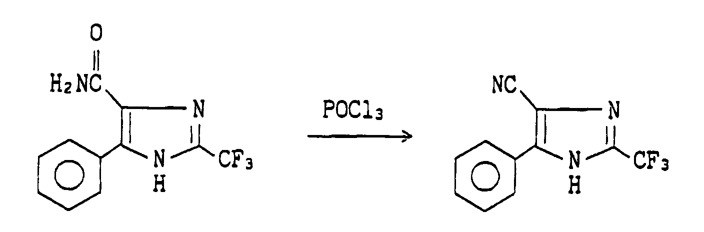

To 19g (0.0203 mol) of 4-carbamoyl-5-phenyl-2-trifluoromethylimidazole was added 30ml of phosphorus oxychloride and heated for 1/2 hours. After cooling the mixture, the mixture was poured into ice-water under stirring at the temperature below 50°C to decompose excess of phosphorus oxychloride. Then, the mixture was extracted by a solvent mixture of 1 to 1 chloroform-ethyl acetate, and was filtered and finally was concentrated under reduced pressure. 4.19g of the crystalline compound targeted was obtained. (yield 87%) m.p. 183-187 °C

### Example 4

### The synthesis of 4-carboxy-5-(4-chlorophenyl)-2-trifluoromethylimi dazole (Compound No. II-59)

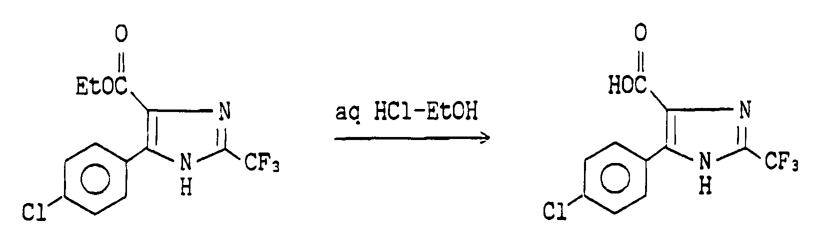

To 1.6g(0.005moles) of 5-(4-chlorophenyl)-4-ethoxycarbonyl-2-trifluoromethylimidazole was added 20ml of of ethyl alcohol and 10ml of 6N-hydrochloric acid. Then the reaction mixture was heated to reflux for three days. After cooling, the reaction mixture was concentrated under reduced pressure and then a solution of caustic soda was added into said obtained residue to make the mixture with strong alkaline property. Said alkaline was extracted with ethyl acetate to recover unreacted material. Cooling the obtained water layer, it was neutralized with dilute hydrochloric acid and was extracted by ethyl acetate, and separated ethyl acetate layer was dried with anhydrous magnesium sulfate. Said solution was filtered and ethyl acetate was removed by vacuum distillation then 0.70g of objective compound was obtained. (yield 49%) d.p. 224°C

### Example 5

### The synthesis of 5-(4-chlorophenyl)-4-N,N-dimethylcarbamoyl-2-trifluoromethylimidazole (Compound No. II-50)

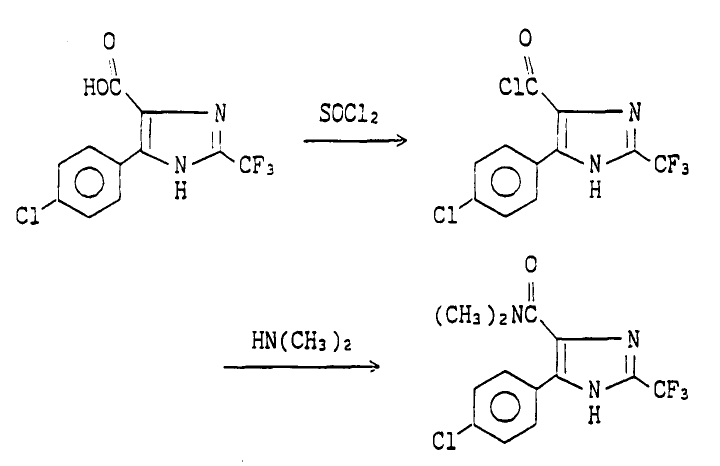

2.47g of 4-carboxy-5-(4-chlorophenyl)-2-trifluoromethylimidazole (0.0085 mol) was added to 10ml of thionyl chloride and was heated to reflux for 1 hour. Excess thionyl chloride was then removed by distillation under reduced pressure and 2.6g of a yellow composition of acid chloride was obtained. Into the mixture of 10ml of water, 20ml of chloroform and 13ml of 50% aqueous solution of dimethylamine, 20ml of chloroform suspension therein obtained 2.6g of crude acid chloride was dispersed, was added dropwise under stirring and ice-cooling. The crude crystalline of said acid chloride was solubilized slowly and after one hour it was disolved completely. To acidify the water layer, diluted hydrochloric acid was added therein. Then, separated chloroform layer was collected and the water layer was extracted with ethyl acetate thoroughly. Two organic layers were combined, and was dried with anhydrous magnesium sulfate, and filtered. The solution was concentrated inder reduced pressure and the obtained crystalline was washed with chloroform. 1.35g of the objective compound was obtained and additionally, 0.4g of the objective compound was recovered by a silica gel column chromatography of wash waste of the chloroform (yield 66%) m.p. 226-227 °C

### Example 6

### The synthesis of 4-bromo-5-(4-chlorophenyl)-2-trifluoromethylimida zole (Compound No. II-88)

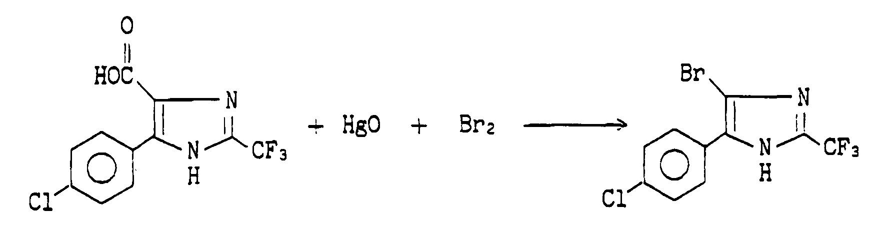

To 2g of 4-carboxy-5-(4-chlorophenyl)-2- trifluoromethylimidazole (0.0069 mol), 1.13g of red mercuric oxide (0.01 mol) and 15ml carbon tetrachloride were added dropwise the solution of 1.1 g bromine in 5ml carbon tetrachloride.

After one hour of refluxing the reaction mixture was filtered and was washed with ethyl acetate and then the filtrates were combined together. The filtrate was washed by a aqueous sodium bicarbonate and the collected organic layer was dried with anhydrous magnesium sulfate and then said solution was filtered and was concentrated under reduced pressure. The residue obtained was disolved in chloroform and insoluble substances was removed by a filtration and after purification by a silicagel columnchromatography 1.2g of the targeted compound was obtained. (yield 54%) m.p. 174-179°C

### Example 7

### The synthesis of 5-(4-chlorophenyl)-4-ethoxycarbonyl-2-methoxycarbonylimidazole (Compound No.II-31)

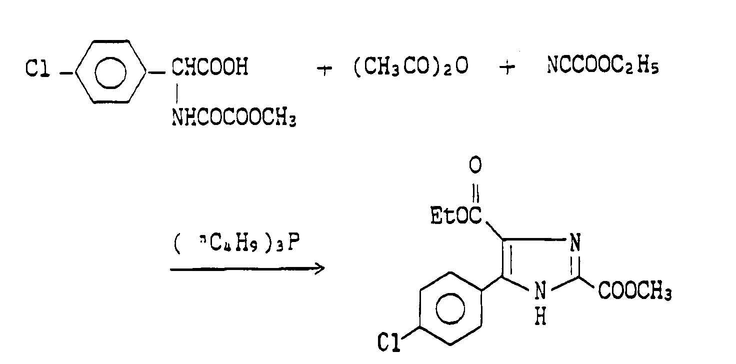

1.36g of 2-(4-chlorophenyl)-N-methyloxalylglycine(0.005 mol), 0.51g of acetic anhydride(0.005 mol) and 0.50g of ethyl cyanoformate disolved in 20 ml of toluene and 1.01g of tributyl phosphine(0.005 mol) was added dropwise with stirring. The mixture was heated gradually and finally was heated at 100°C for one hour. After cooling said mixture, it was concentrated under reduced pressure. Said residue was purified using a silicagel columnchromatography and 0.9g of objective compound was obtained. (Yield 58%) m.p.149-151°C

### Example 8

### The synthesis of 5-(3-chlorophenyl)-4-ethoxycarbonyl-2-methylimidazole (Compound No.II-16)

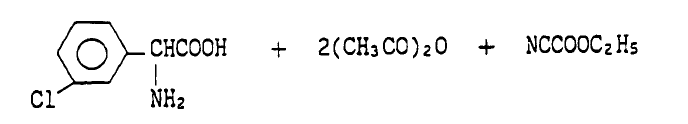

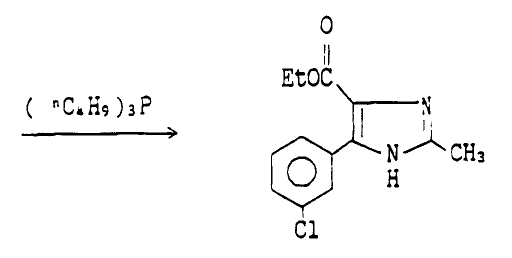

1.86g of 2-(3-chlorophenyl) glycine (0.01 mol), 2.04 g of acetic anhydride(0.02 mol) and 0.99g of ethyl cyanoformate (0.01 mol) were disolved in 10ml of toluene and with stirring into said reaction mixture 2.02g of tributyl phosphine was added dropwise. The reaction mixture was gradually heated and was kept for 30 minutes under refluxing and was concentrated under reduced pressure. Said residue was purified using a silicagel columnchromatography with a solution of 20 to 1 chloroform-methanol. 1.53g of the targeted compound was obtained.
(Yield 58%) nD ^{23.01} 1.5576

### Example 9

### The synthesis of 5-(3,4-dichlorophenyl)-2-trifluoromethylimidazole

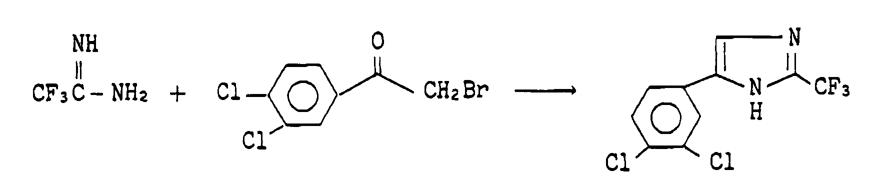

Into the mixture of 22.4g of trifluoroacetamidine (0.2 moles), 160ml of dimethylformamide and 160ml of acetone, 21.5g of 3,4-dichlorophenyl bromide(0.08mol) was added dropwise at room temperature and said reaction mixture was kept under stirring for 24 hours. The acetone was removed from said mixture under reduced pressure and said mixture was poured into 500ml of ice-water ant the resultant was extracted with 500ml of mixed solvent of 1 to 1 benzene-ethyl acetate. The obtained organic layer was washed with water and was dried with anhydrous magnesium sulfate. Further the residue obtained was purified by silicagel columnchromatography. 16.5g of purified 5-(3,4-dichlorophenyl)-2-trifluoromethylimidazole was obtained. (yield 73.5%) m.p.179-180°C

### Example 10

### The synthesis of 4-bromo-5-(3,4-dichlorophenyl)-2-trifluoromethyli midazole (Compound No.II-108)

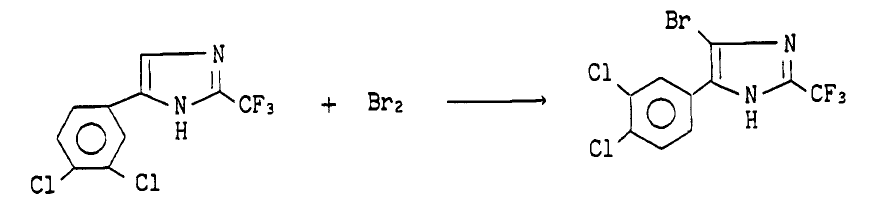

1.99g of 5-(3,4-dichlorophenyl)-2-trifluoromethylimidazole(7.08mmo l) was dissolved into 35ml of chloroform and into this mixture under ice-cooling, 1.36g of bromine (8.5 mol) in 10ml of chloroform solution was added dropwise. After keeping said mixture under stirring for one hour, it was poured into water. Separated chloroform phase was collected and was washed by the water solution containing sodium bisulfite. After drying and concentrating, said residue was purified using a silicagel chromatography to give 1.91g of the objective compound.
(yield 75%) m.p.201-202 °C

### Example 11

### The synthesis of 4-chloro-5-(3,4-dichlorophenyl)-2-trifluoromethylimidazole (Compound No.II-149)

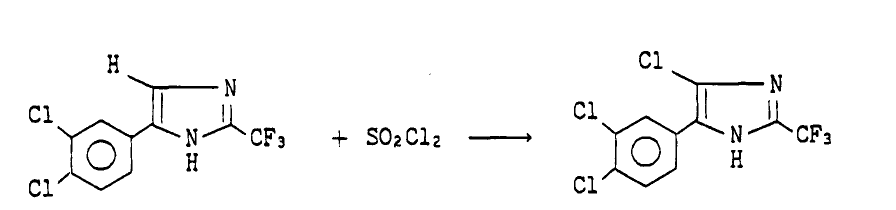

1.70g of 5-(3,4-dichlorophenyl)-2-trifluoromethylimidazole (6.05 mmoles) was dissolved in 60ml of acetic acid and was added 0.9g of sulfuryl chloride dropwise at room temperature. After stirring at room temperature for 2 hours, said mixture was poured into ice-water and was extracted with the mixed solvent of 1 to 1 ethyl acetate-benzene. Obtained organic layer was washed with water and aquous sodium bisulfite. After drying and concentrating, said residue was purified by a silicagel column chromatography and 1.56g of the objective compound was obtained. (yield 82%) m.p.192-193°C

### Example 12

### The synthesis of 5-(3,4-dichlorophenyl)-4-iodo-2-trifluoromethylim idazole (Compound No.II-156)

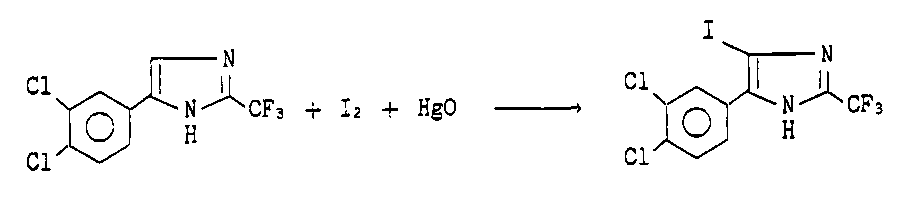

Into the solution in which 1.23g of 5-(3,4-dichlorophenyl)-2-trifluoromethylimidazole (4.4 mmol) was dissolved in 150ml of chloroform, 1.12g of iodine (4.4 mmol) and 0.79g of red mercuric oxide (3.6 mmoles) were added successively and the mixture was kept at room temperature for 3 hours under stirring.

The reaction mixture was filtered and the filtrate was washed with aqueous sodium bisulfite, dried and concentrated. The residue was purified by a silicagel columnchromatography. 1.7g of the compound targeted was obtained. (yield 95%) m.p.201-202 °C

### Reference 1

### The synthesis of 2-(4-chlorophenyl)-5-(3-trifluoromethylphenyl) imidazole

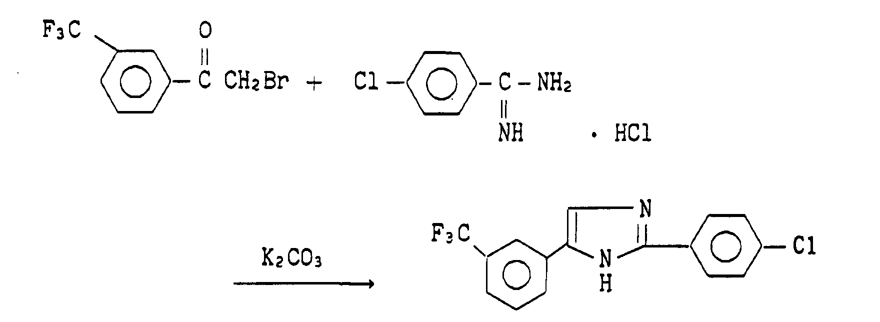

7.54g of 3-trifluoromethylphenacyl bromide (0.028 moles) was added dropwise into the mixture in which 6g of 4-chlorobenzamidine (0.032 mol) was dissolved in the solvent mixture of 70ml to 100ml dimethylformamide -acetone, and into said mixture 22.5g of potassium carbonate (0.16 mol) was added under stirring and was kept at room temperature under stirring. The reaction mixture was poured into 500ml of ice-water and was extracted with 400ml of the mixed solvent of 1 to 1 benzene-ethyl acetate. The organic layer was washed with water and then dried with anhydrous magnesium sulfate, it was concentrated. Said residue was purified by a silicagel columnchromatography and 4.08g of 2-(4-chlorophenyl)-5-(3-trifluoromethylphenyl)imidazole was obtained. (yield 45%) m.p.178-179 °C

### Example 13

### The synthesis of 2-(4-chlorophenyl)-4-nitro-5-(3-trifluoromethylph enyl)imidazole (Compound No.II-162)

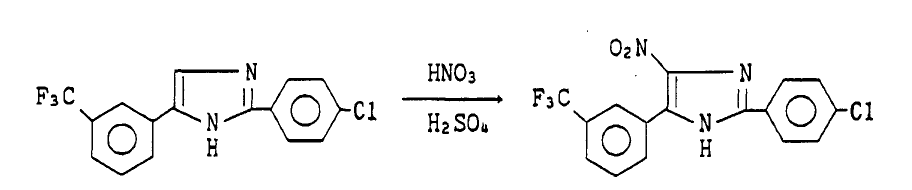

1.03g of 2-(4-chlorophenyl)-5-(3-trifluoromethylphenyl) imidazole(3.2 mmol) was mixed with 40ml of ice-cooled concentrated sulfuric acid. And 0.29g of 70% conc. nitric acid was added dropwise into said mixture gradually at 0 °C and said mixture was warmed slowly to room temperature and was kept under stirring for 3 hours. The reaction mixture was poured into 100ml of ice-water and was extracted with ethyl acetate and the organic layer separated was washed with water and dried with anhydrous magnesium sulfate. After concentrating said solution, obtained crude crystallines was washed by ether. 0.88g of 2-(4-chlorophenyl)-4-nitro-5-(3-trifluoromethylphenyl) imidazole was obtained. (yield 75%) m.p.253-255°C

### Example 14

### The synthesis of 1-acetyl-4 (5)-bromo-(4-chlorophenyl)-5(4)-phenylimidazole (Compound No.II-161,I-98)

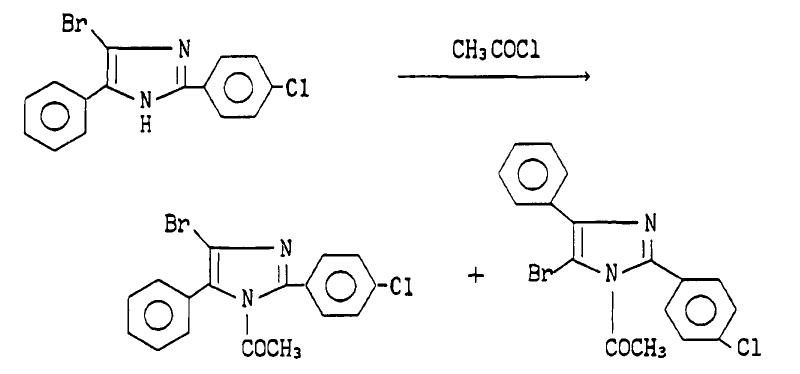

Into the solution in which 1.0g of 4-bromo-2-(4-chlorophenyl)-5-phenylimidazole (0.003 mol) was dissolved in 30ml of dimethylformamide, 0.68g of triethylamine (0.0067 mol) was added and the mixture was under stirring for 5 minutes. Into said mixture 0.52g of acetyl chloride (0.0067 mol) was added dropwise and further was kept under stirring for 4 hours at room temperature. Said mixture was poured into 100ml of ice-water and was extracted by 150ml of ethyl acetate and the layer thereof was washed with water and dried over anhydrous magnesium sulfate. After concentrating said solution, the crystallines obtained was washed by the mixed solvent of hexane-ether and 0.76g of 1-acetyl-4-bromo-2-(4-chlorophenyl)-5-phenylimidazole was obtained. (yield 76%) m.p.114-116°C

In addition, after the concentration of said hexane-ether washing solution, and after the purification of obtained residue by a silicagel columnchromatography, 1-acetyl-5-bromo-2-(4-chlorophenyl)-4-phenylimidazole was obtained. (yield 7%) m.p.130-131°C

### Example 15

### The synthesis of the compounds wherein X is H and R is -CH₂OC₂H₅

### The synthesis of 5-ethoxycarbonyl-1-ethoxymethyl-4-phenyl-2-trifluoromethylimidazole and 4-ethoxycarbonyl-1-ethoxymethyl-5-phenyl-2-trifluoromethylimidazole (Compound No.I-24 and II-46)

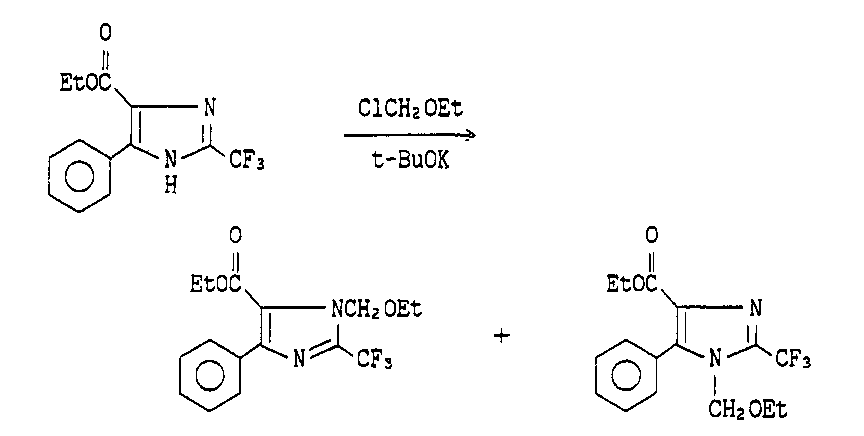

2.0g of 2-ethoxycarbonyl-5-phenyl-2-trifluoromethylimidazole (0.007 mol) was dissoved in 70ml of dry tetrahydrofuran and into it 1.18g of potassium-t-butoxide (0.0105 mol) was added and was kept under stirring for 10 minutes at room temperature. Into said mixture 1.0g of chloromethyl ethyl ether (0.0106 mol) was added dropwise and was kept under stirring for 24 hours at room temperature. Said mixture was poured into water and was extracted with ethyl acetate. Separated layer of ethyl acetate was dried over anhydrous magnesium sulfate and was filtered and said filtrate was concentrated under reduced pressure. The obtained residue was purified by a silica gel column chromatography with solvent of 9 to 1 benzene-ethyl acetate. Two compounds were obtained.
5-ethoxycarbonyl-1-ethoxymethyl-4-phenyl-2-trifluoromethylimidazole , 1.47g. (yield 61%) nD^{23.3} 1.5004
4-ethoxycarbonyl-1-ethoxymethyl-5-phenyl-2-trifluoromethylimidazole , 0.44g. (yield 18%) nD^{24.0} 1.4946
In the mass spectrometric analysis of the compound of No.II-46, said structure was confirmed from the result of the observation of NOE between the proton of position 1 in N-CH₃ and the proton of ortho position in benzene ring.

### Example 16

### The synthesis of 1-acetoxymethyl-4-(3,4-dichlorophenyl)-5-ethoxycarbonyl-2-trifluoromethylimidazole (Compound No.I-80)

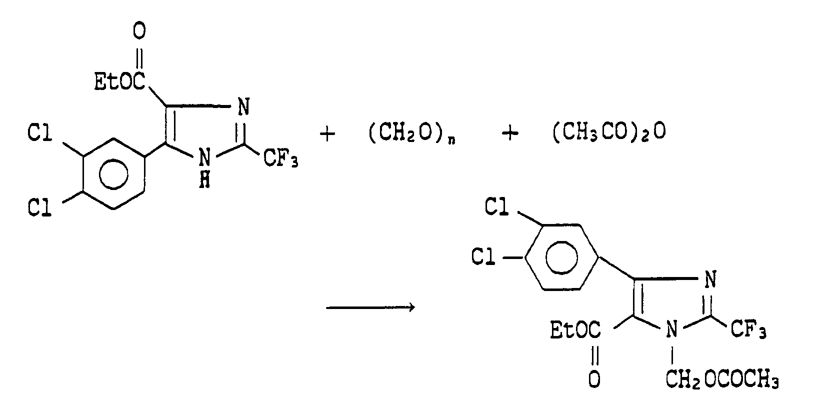

7.06g of 5-(3,4-dichlorophenyl)-4-ethoxycarbonyl-2-trifluoromethylimidazole (0.02 mol), 20ml of acetic anhydride, 0.83g of paraformaldehyde and 0.52g of p-tolenesulfonic acid were mixed together and said mixture was heated under refluxing for 26 hours. The resultant mixture was poured into ice-water and was extracted with mixed solvent of 1 to 1 ethyl acetate-benzene. The obtained organic layer was washed with water and further washed with a water solution of sodium bicarbonate. After drying and concentrating said solution, obtained residue was purified by silicagel column chromatography and 1.83g of the objective compound was obtained. (yield 21%) nD^{24.8} 1.5286
The typical representaives of the compounds of the present invention are demonstrated in table I-I and I-II.

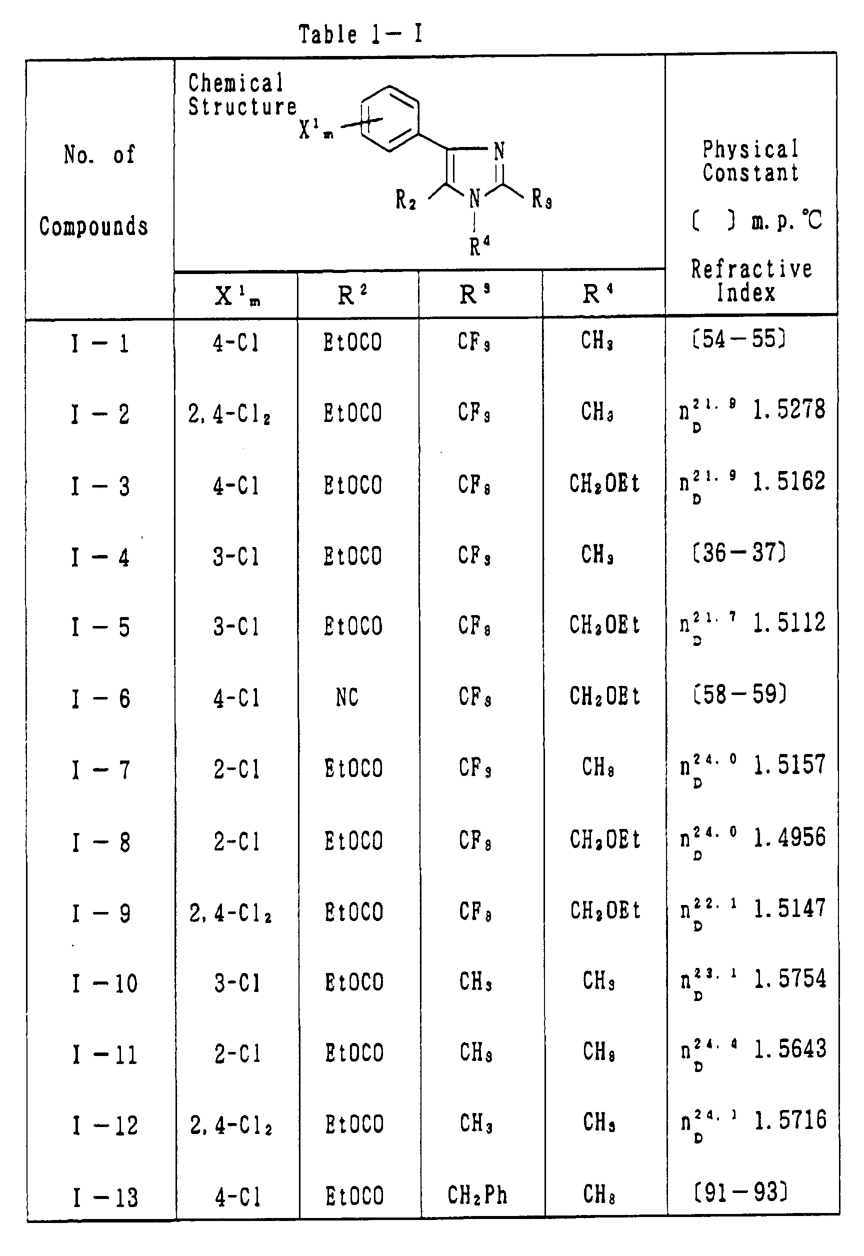

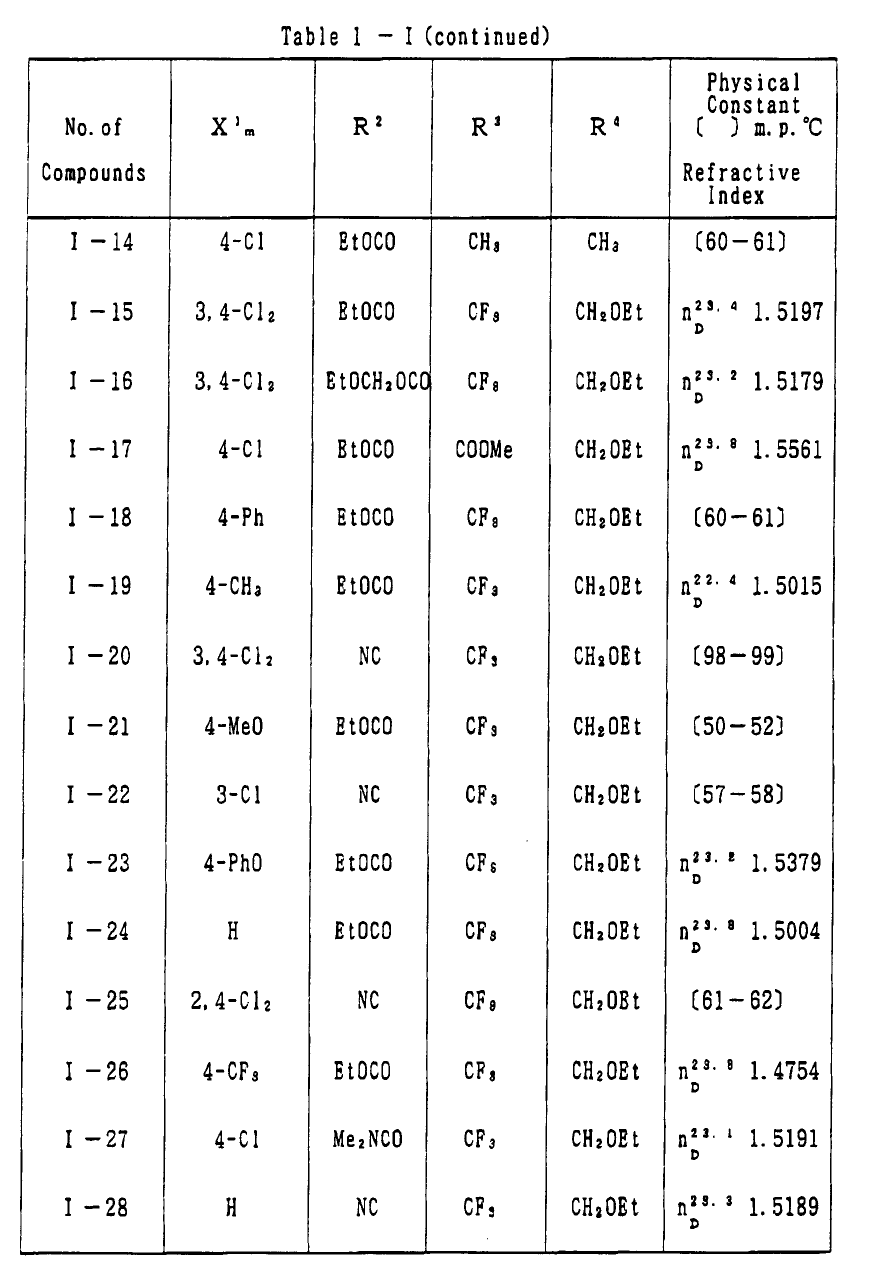

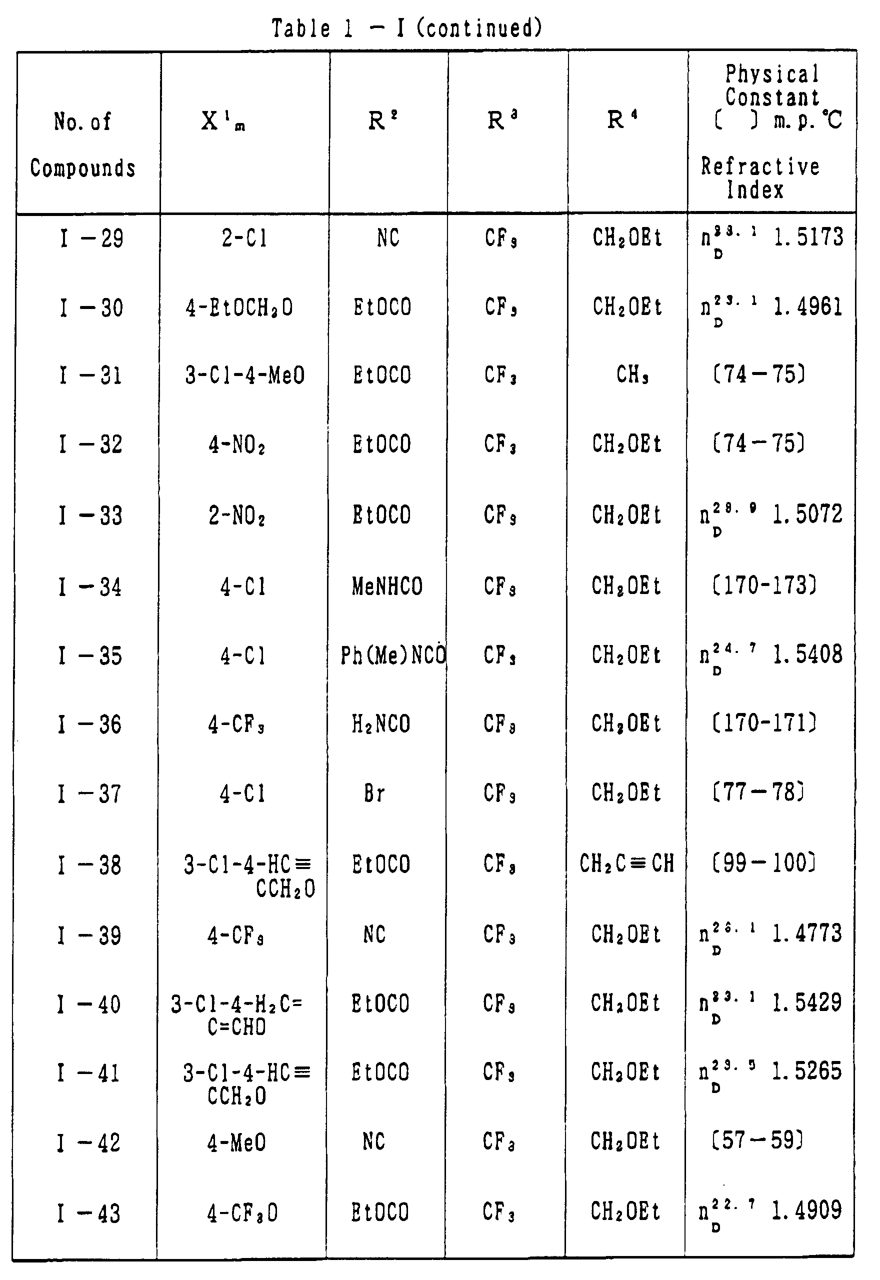

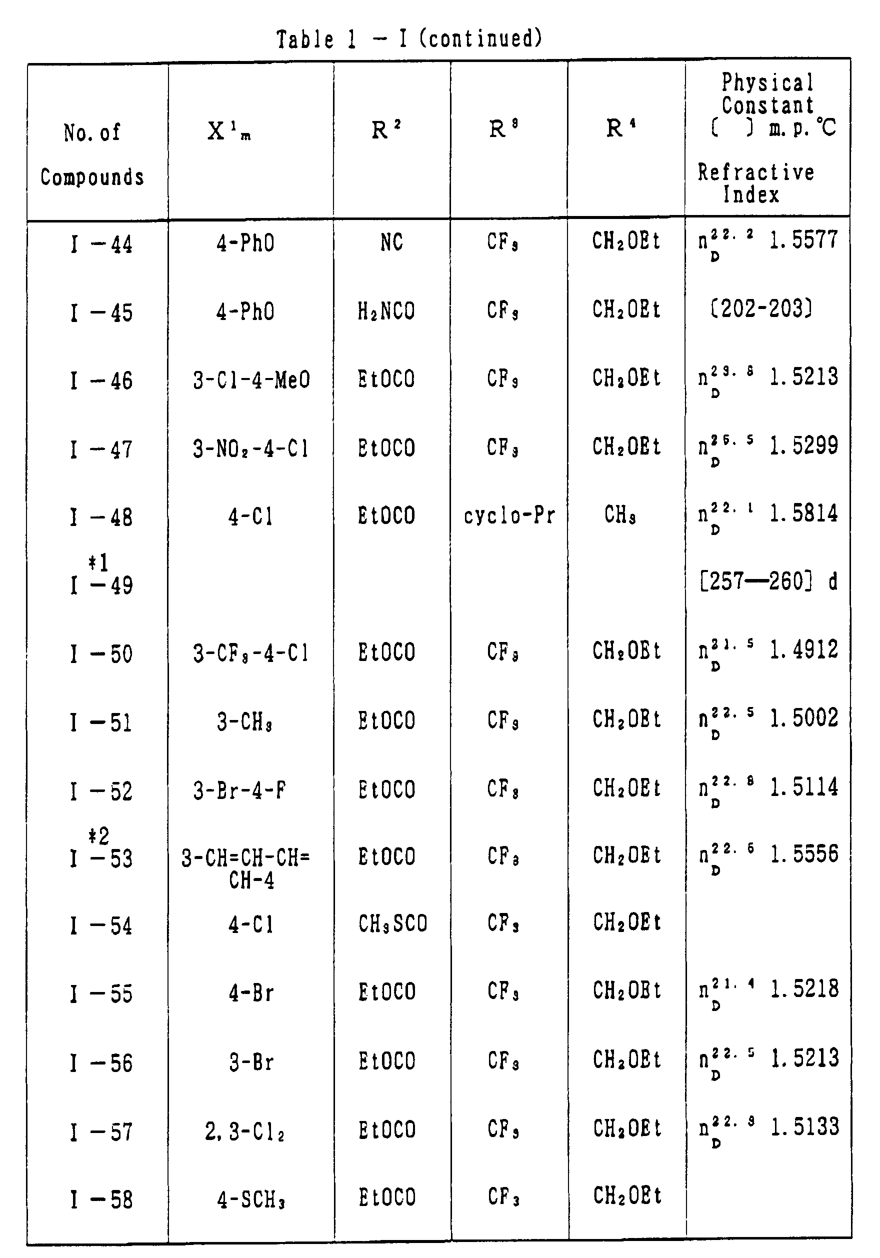

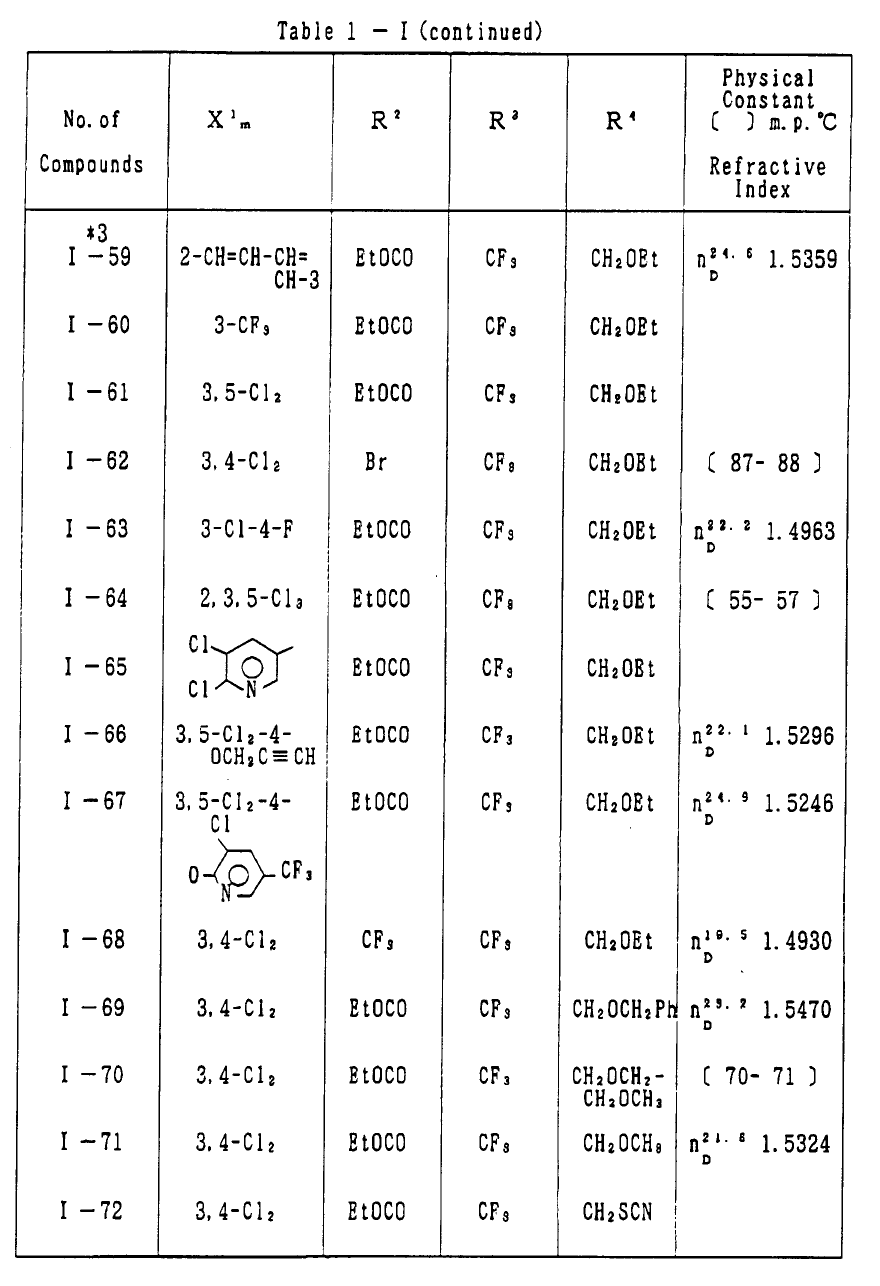

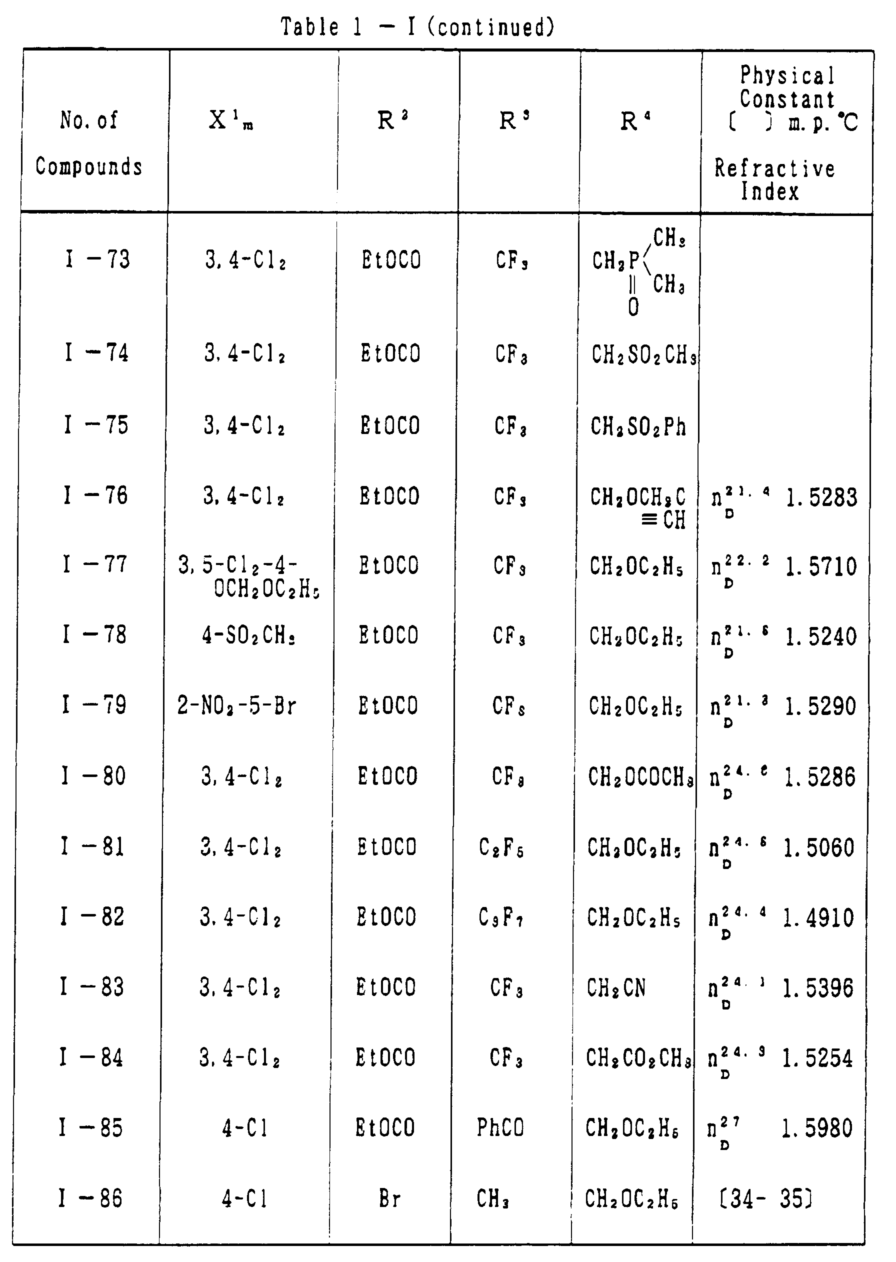

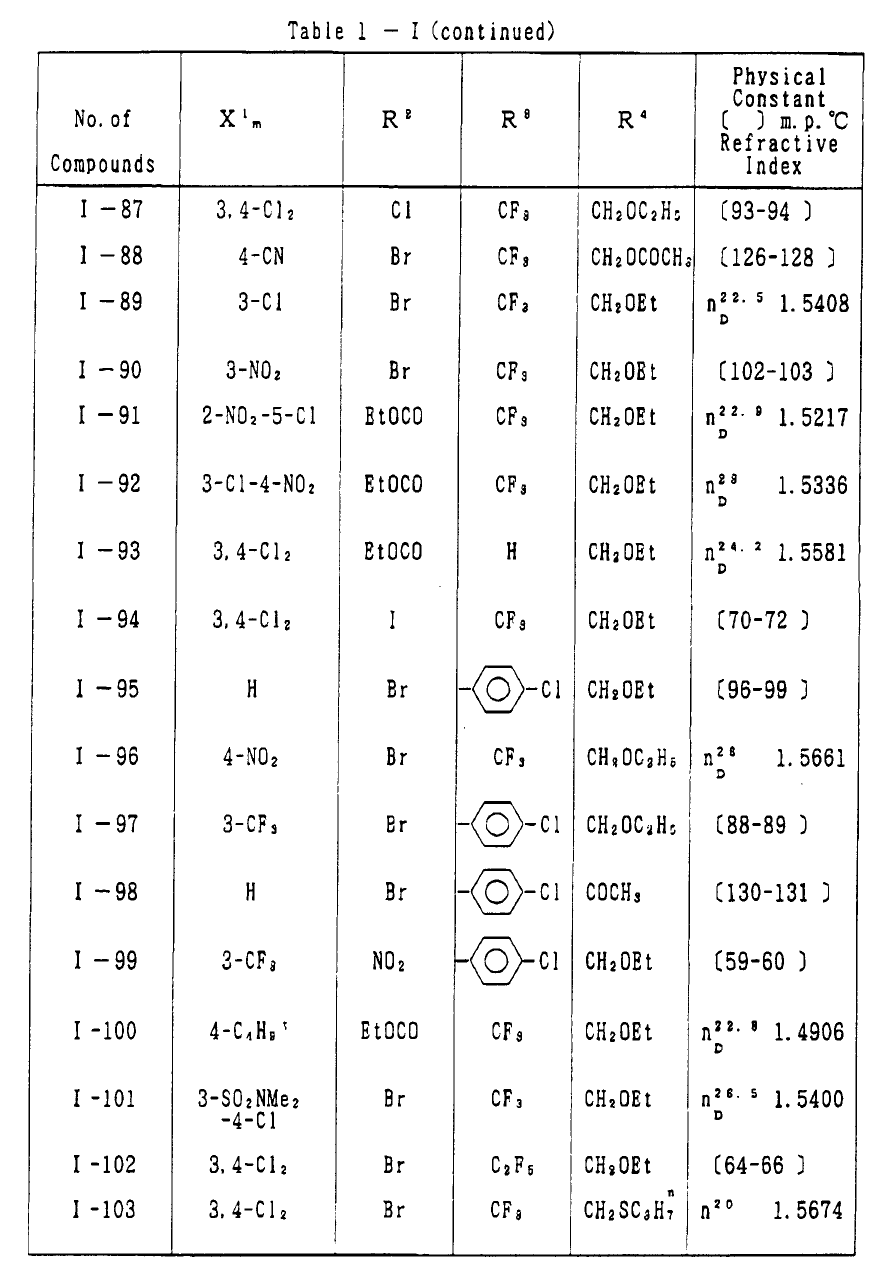

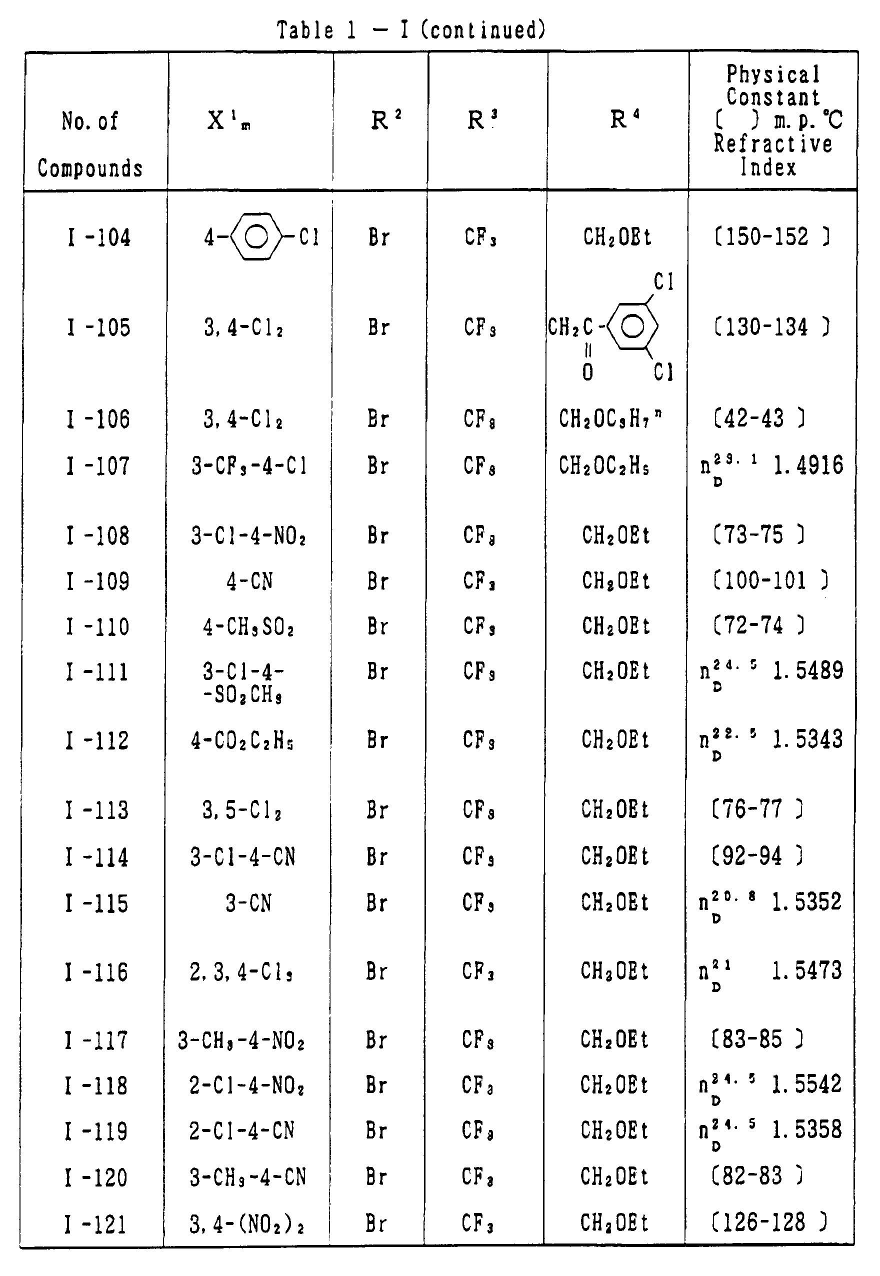

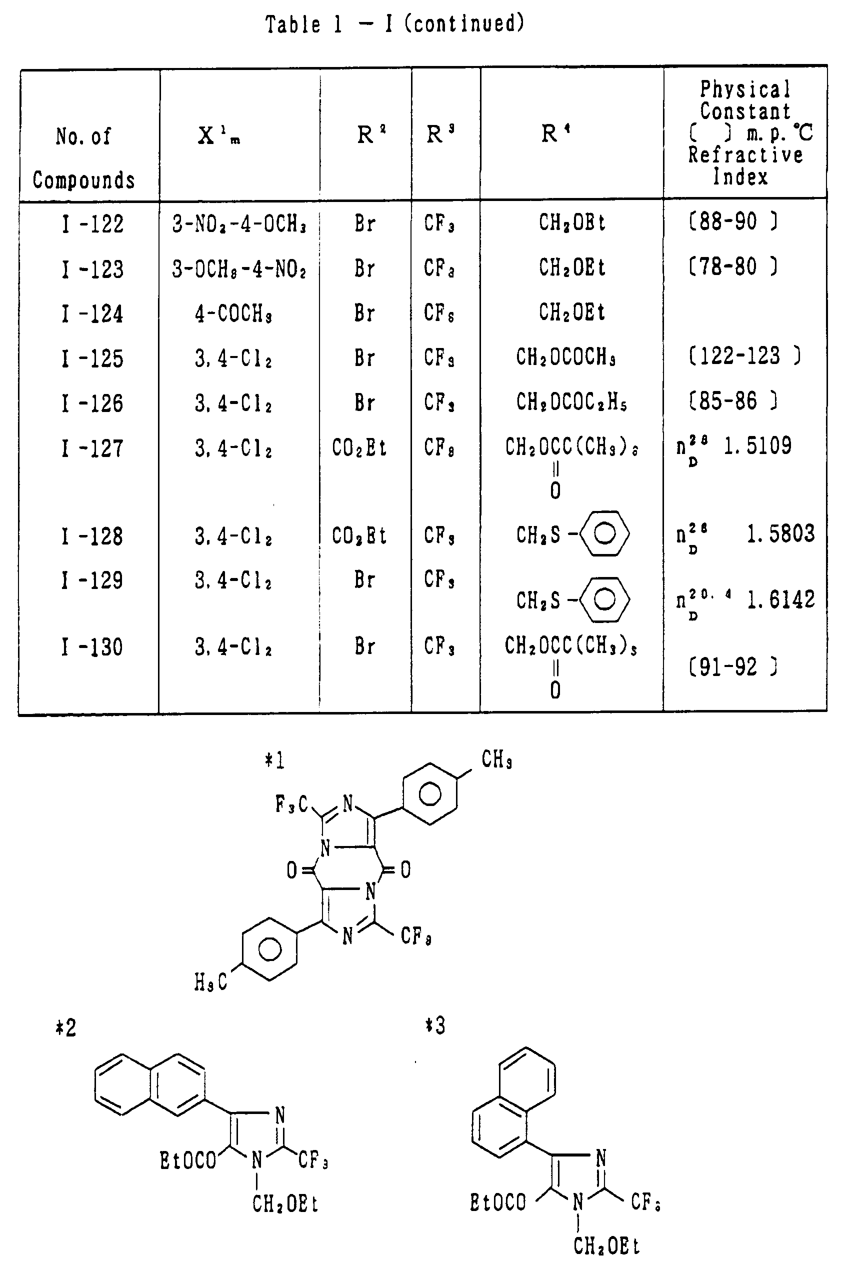

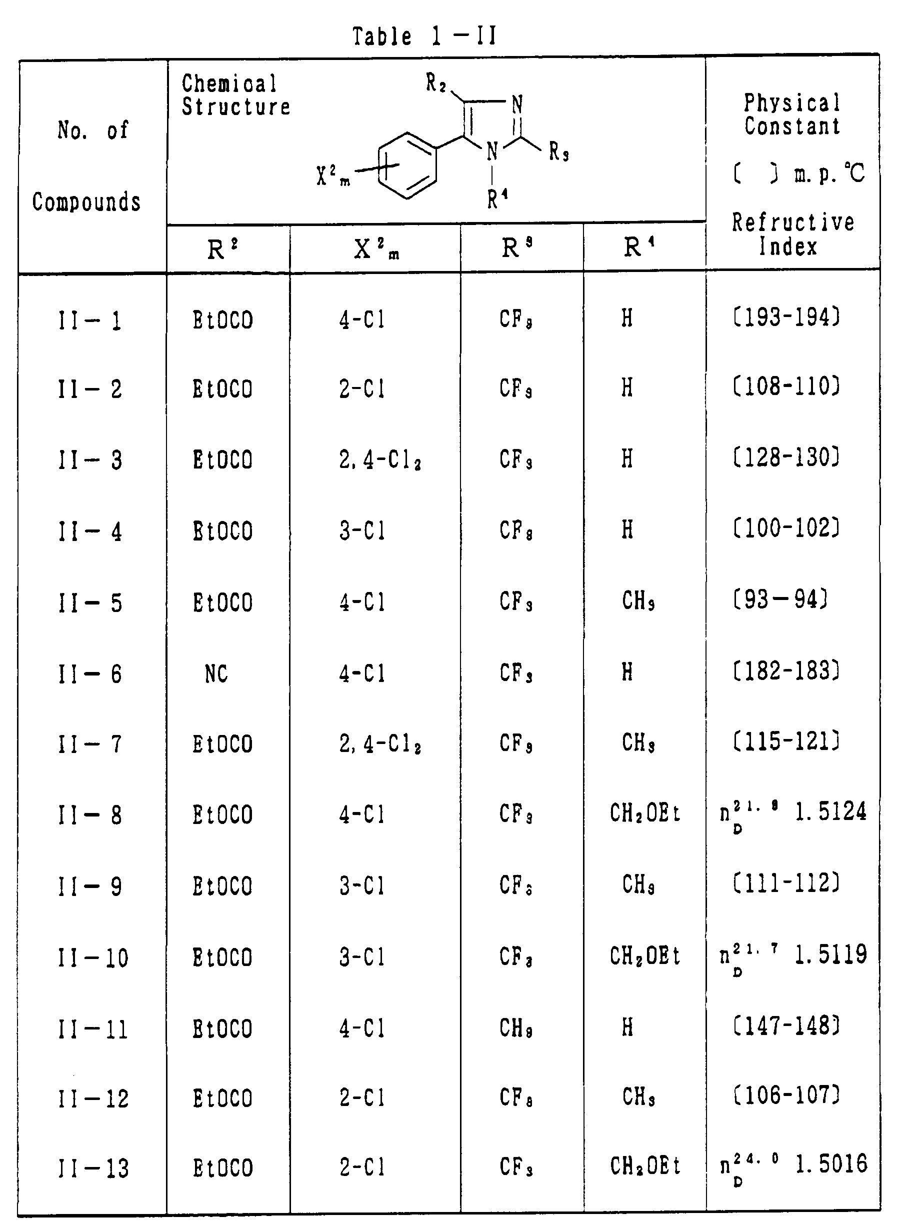

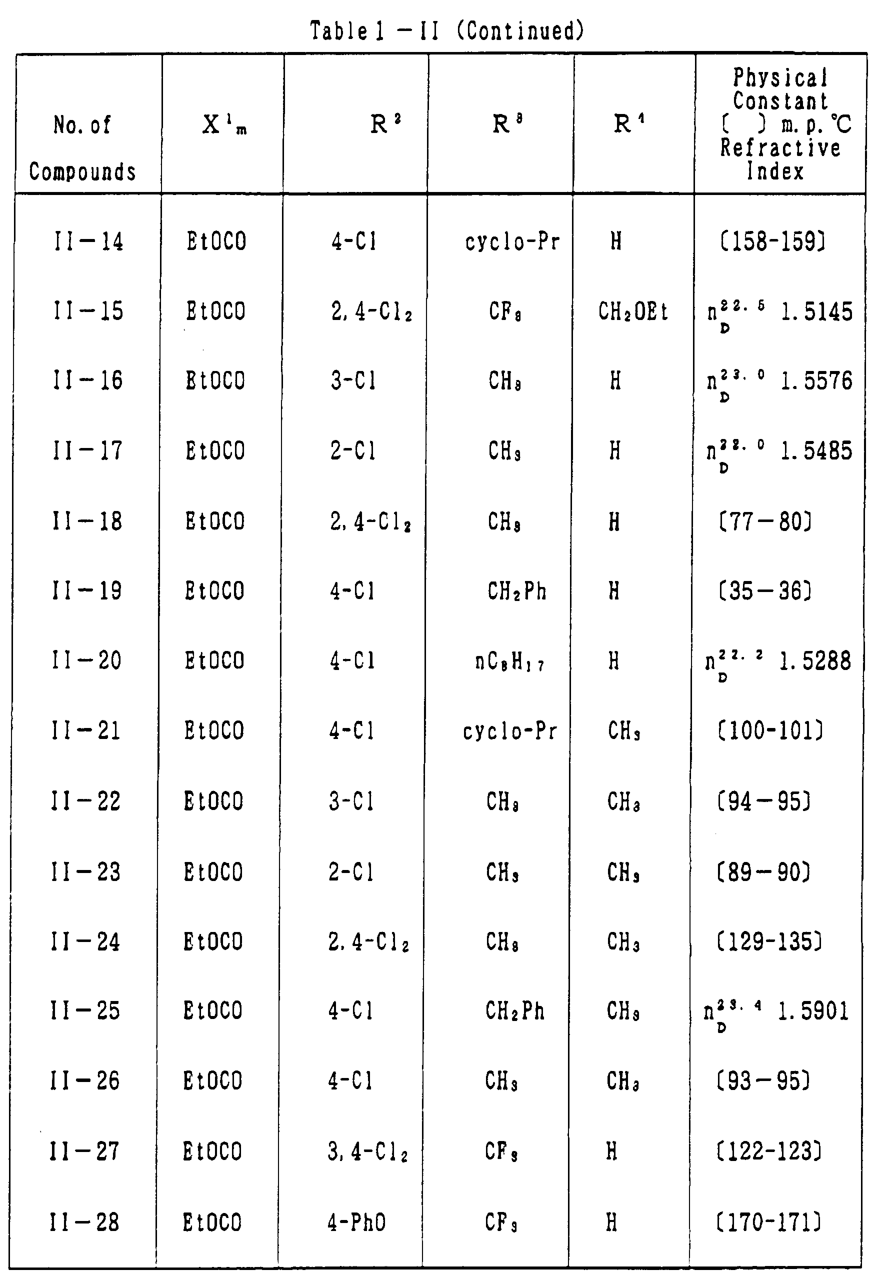

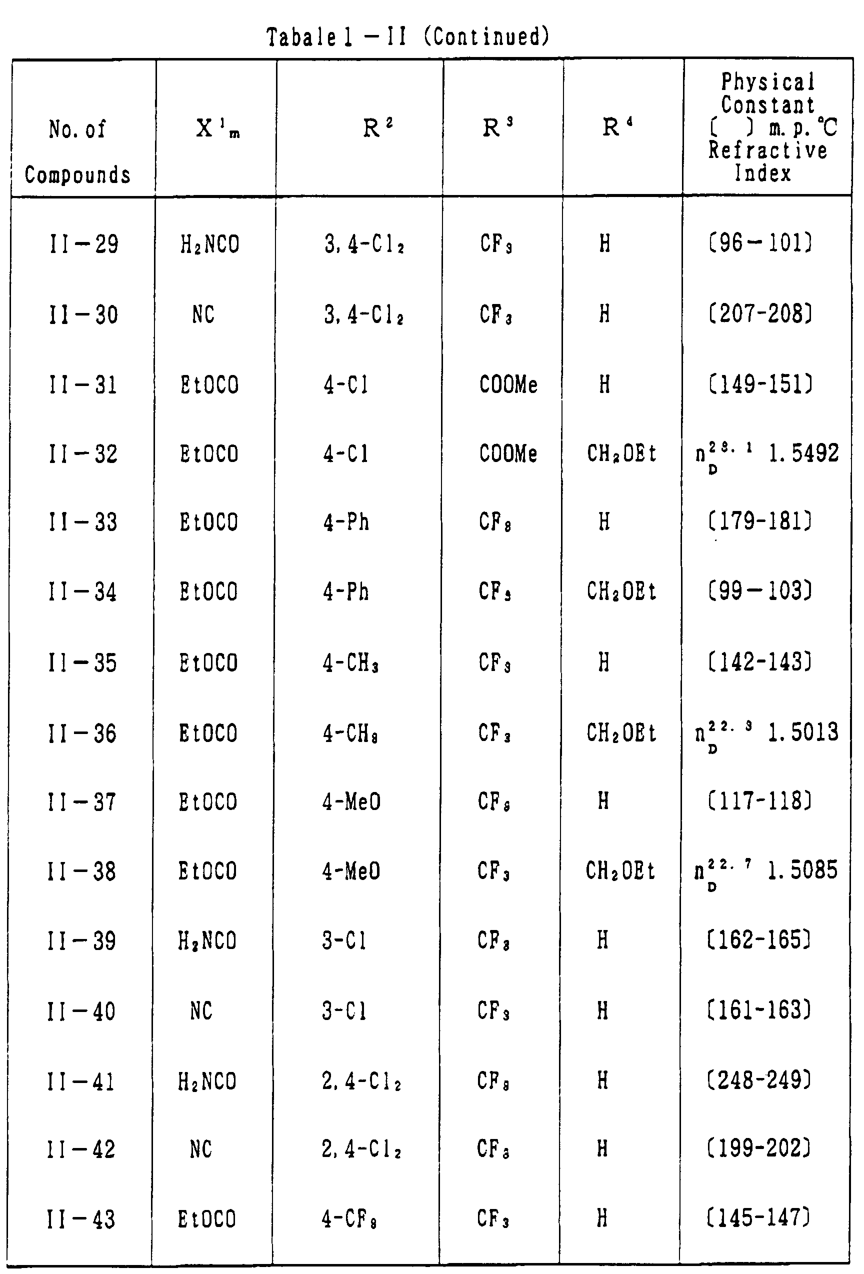

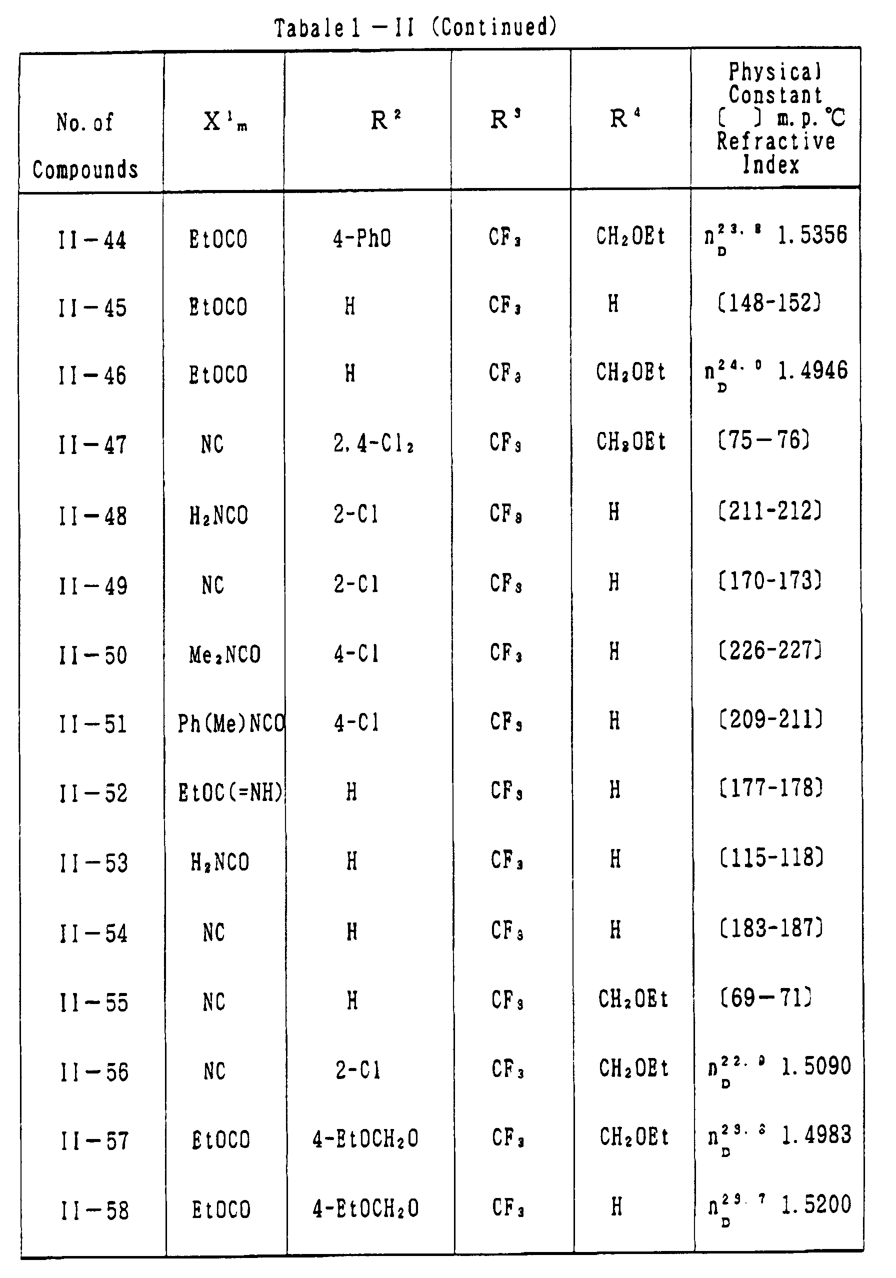

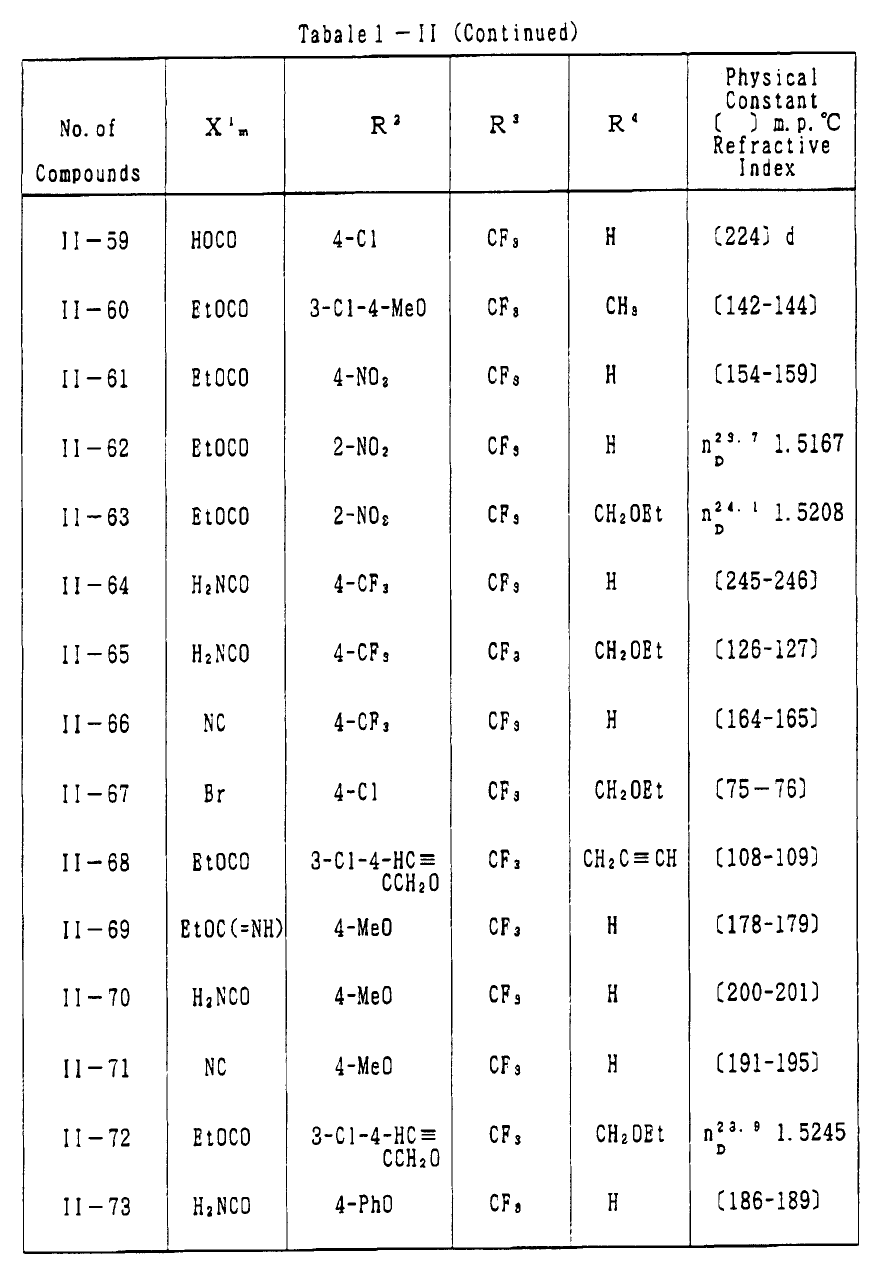

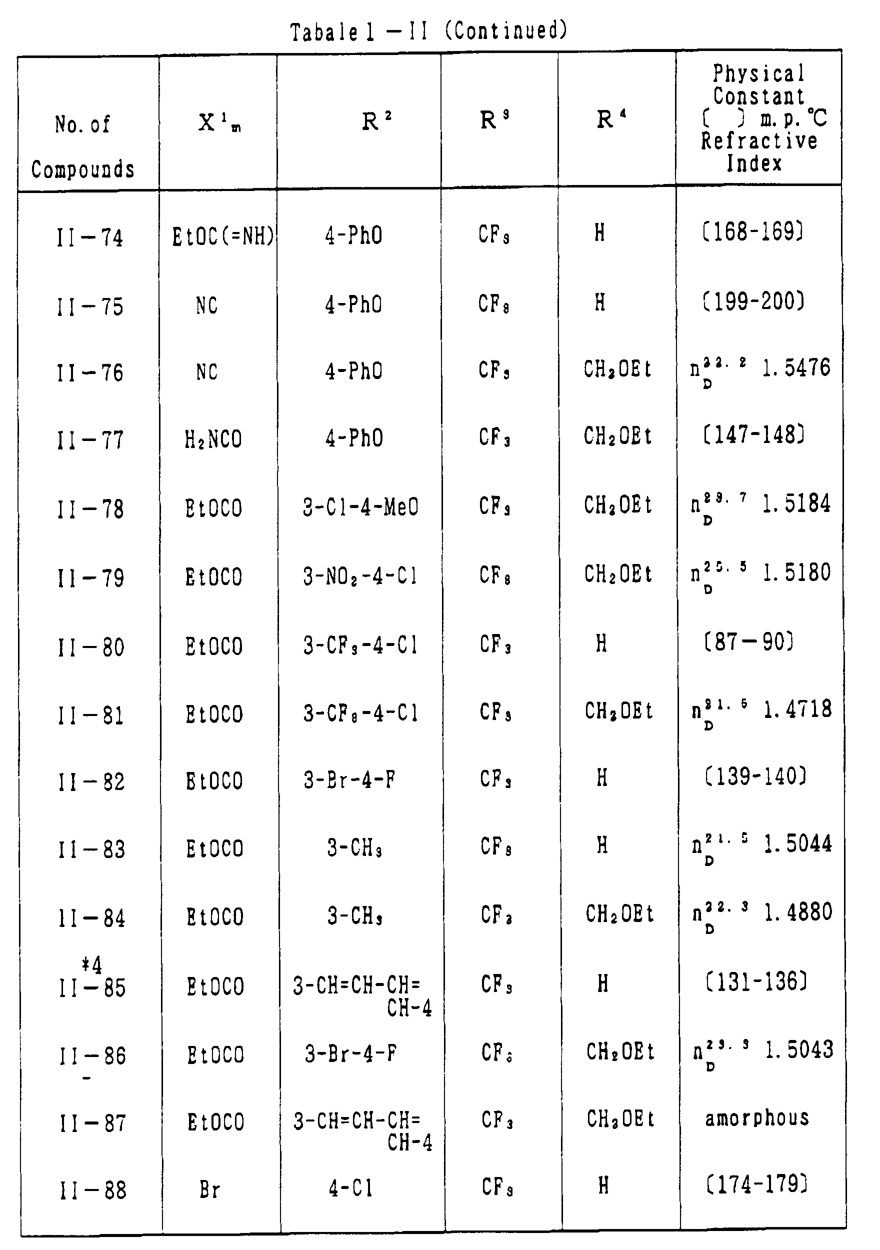

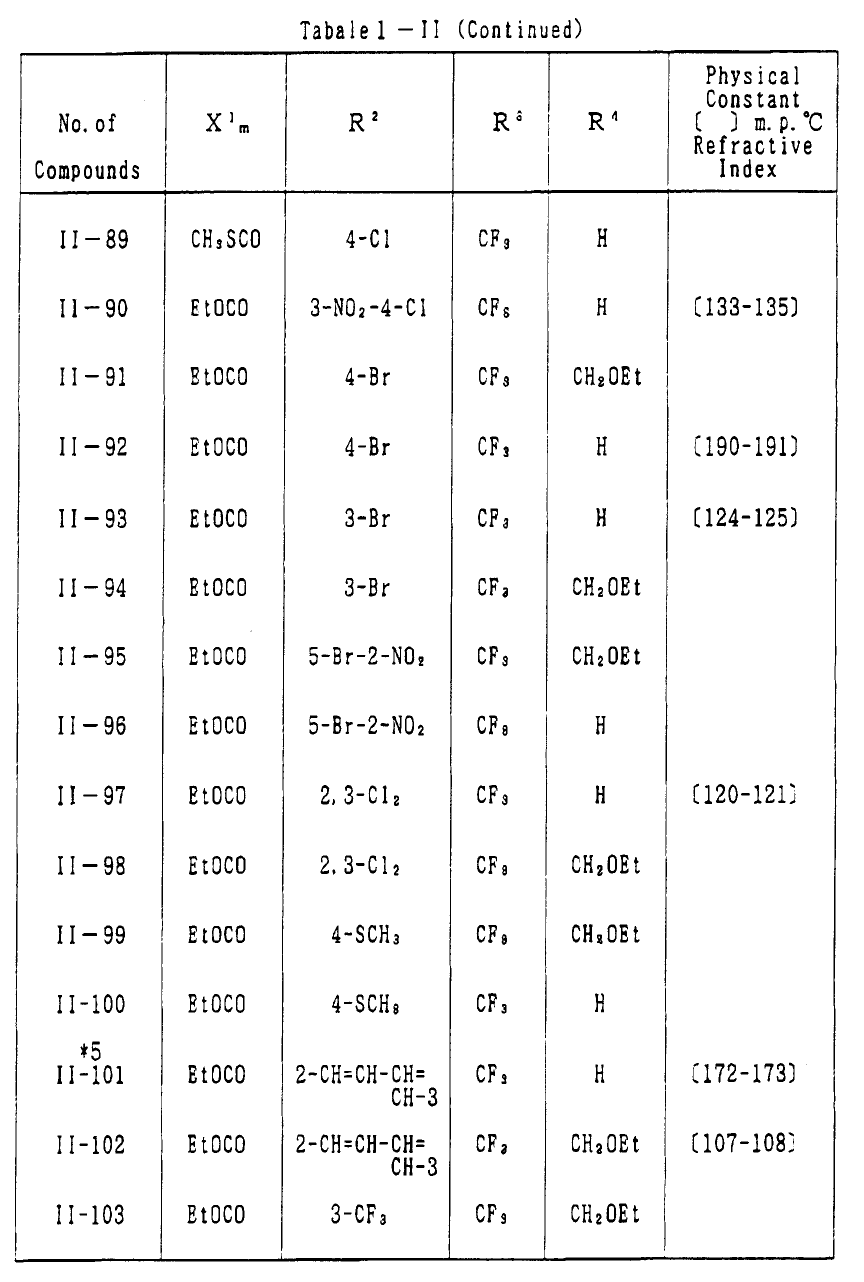

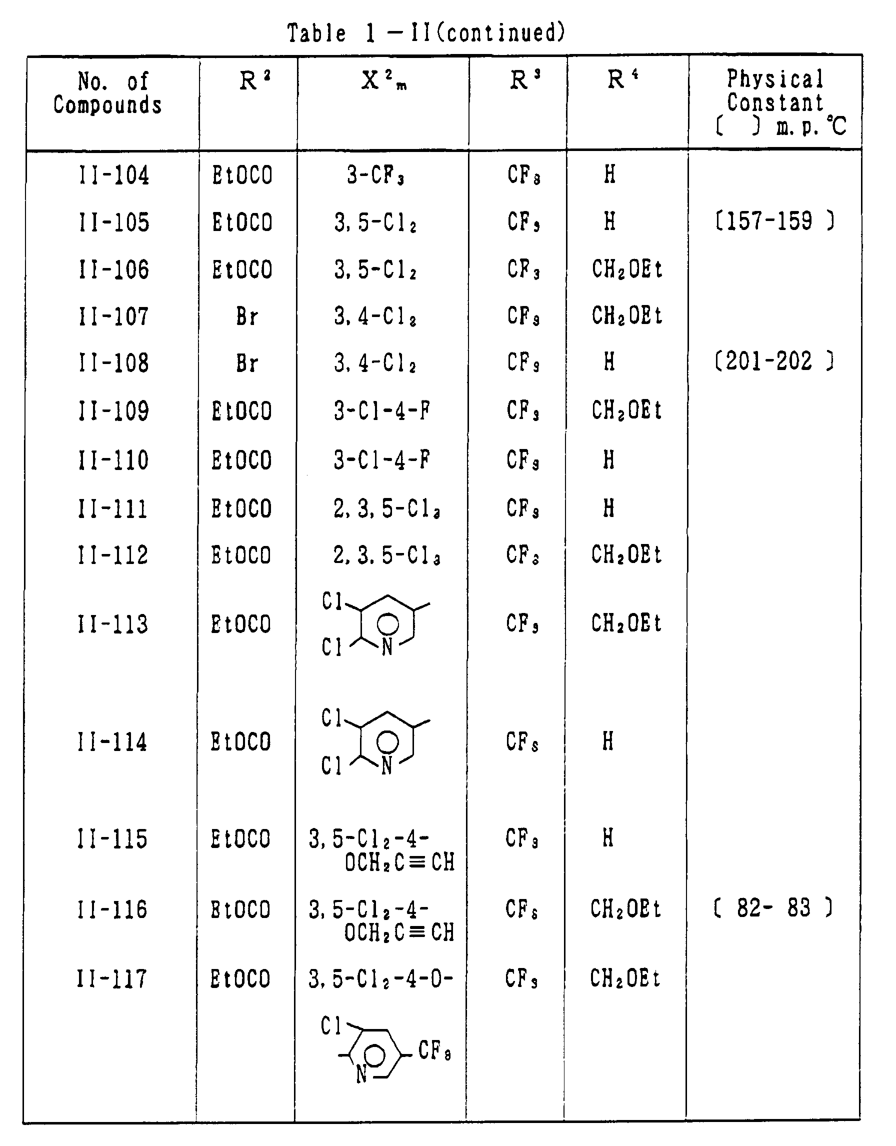

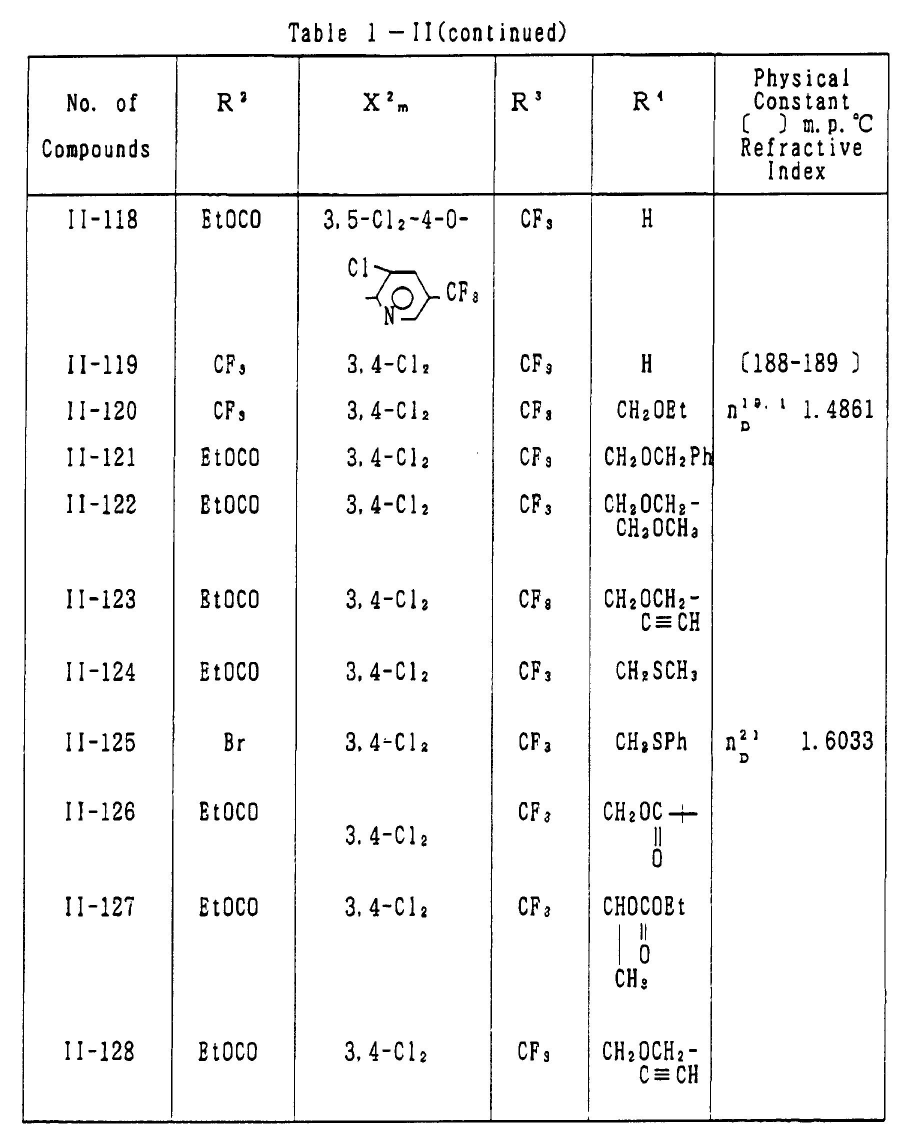

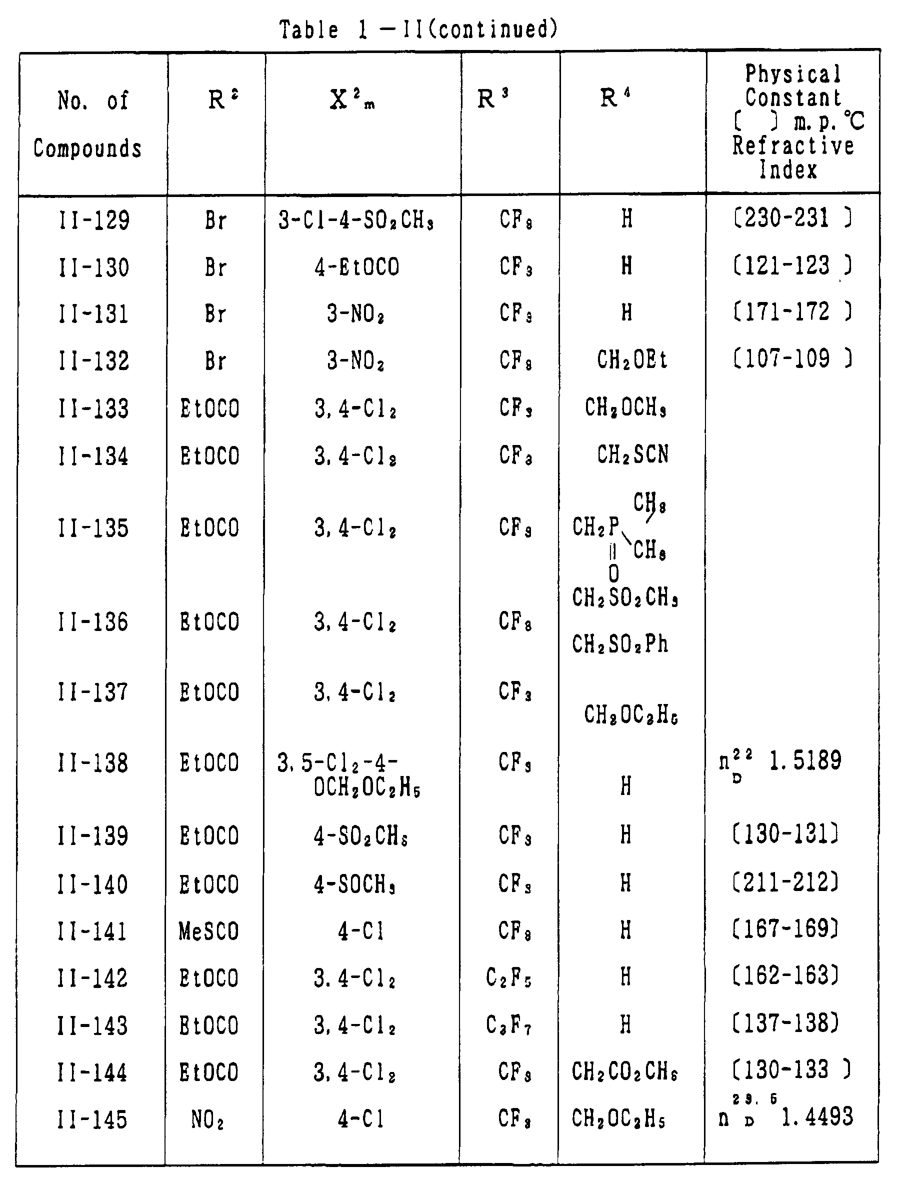

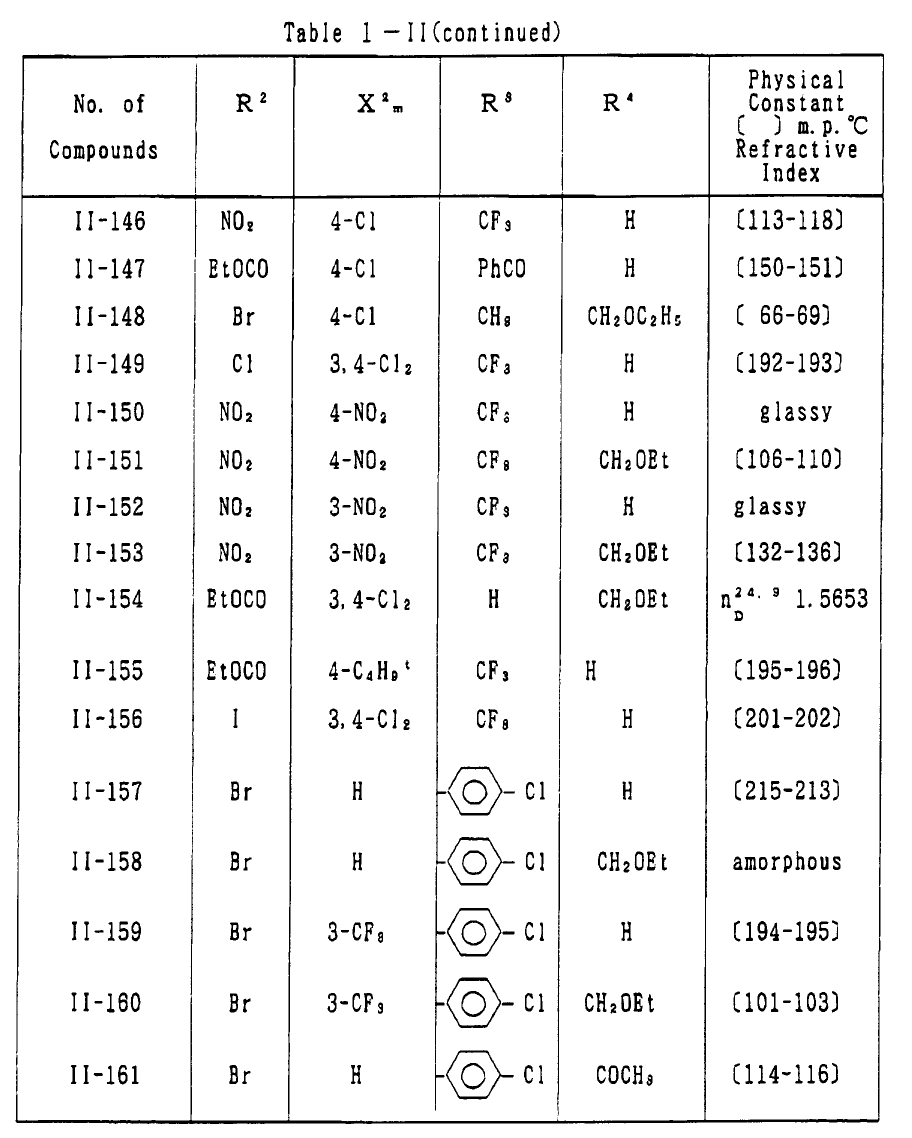

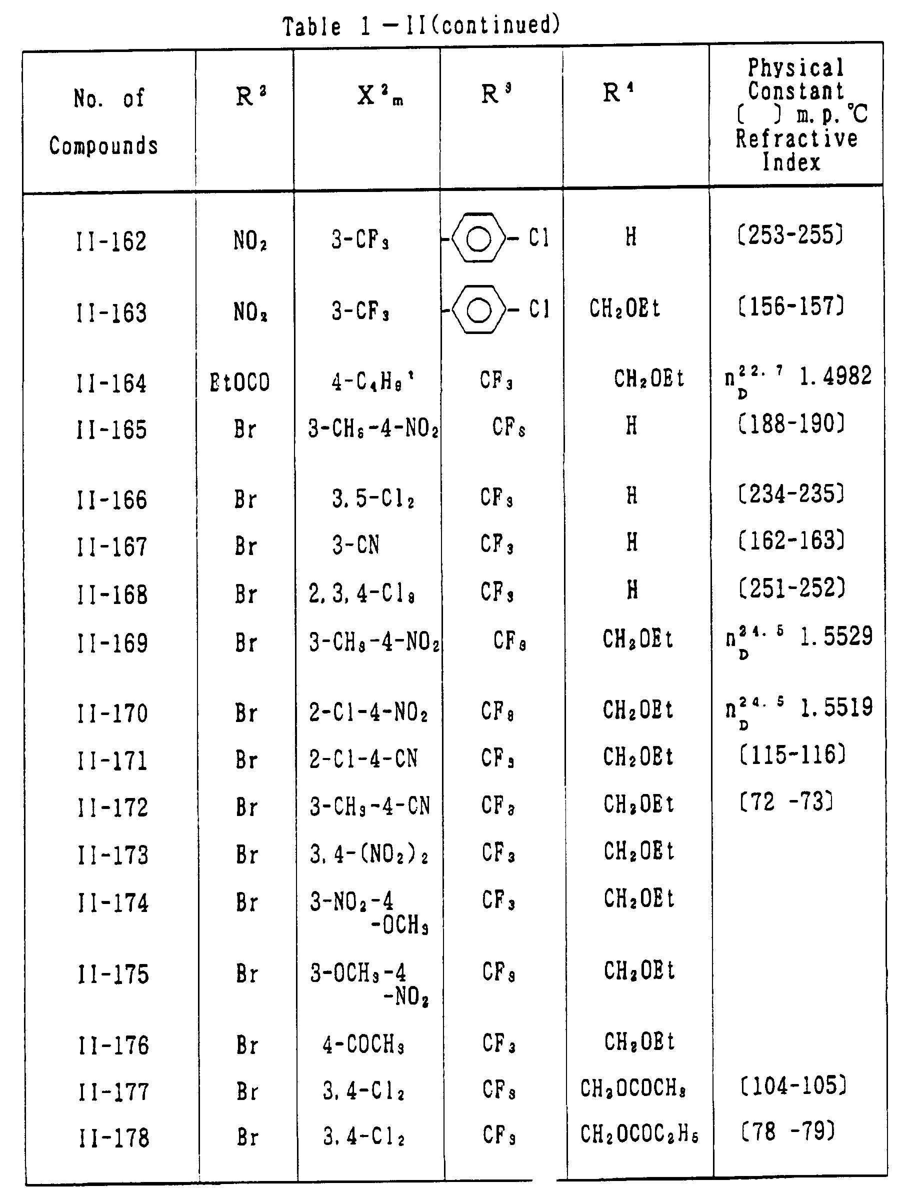

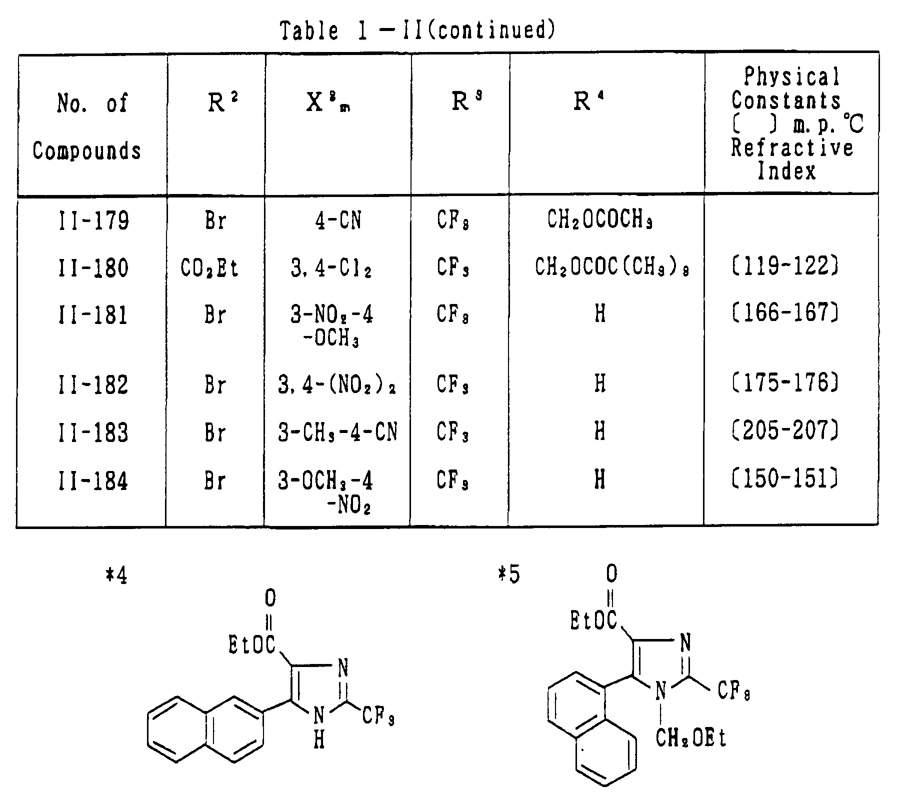

The compounds of the present invention can be used for the control of pests against agricultural production, hygienic pest insects, pest insects in storing grains, pest insects of clothing, house pest insects, etc. The representative examples for such pests are exemplified hereinbelow.

For examples of Lepidopterous pest insects, cotton leafworm, cabbage armyworm, black cutworm, common cabbageworm, cabbage looper, diamond-back, smaller tea tortrix, tea leaf roller, peach fruit moth, oriental fruit moth, citrus leaf miner, tea leaf roller, apple leaf miner, gypsy moth, tea tussock moth, rice stem borer, grass leaf roller, European corn borer, fall webworm, almond moth, Heliothis sp., Helicoverpa sp., Agrotis sp., casemaking clothes moth, codling moth, and cotton bollworm are exemplified. For examples of Hemipterous pest insects, green peach aphid, cotton aphid, turnip aphid, grain aphid, bean bug, common green stink bug, arrowhead scale, mulberry mealy scale, greenhouse whitefly, tobacco whitefly, pear psylla, Japanese pear lace bug, brown planthopper, small brown planthopper, white-backed planthopper, and green rice leafhopper are exemplified. For examples of Coleopterous pest insects, striped flea beetle, cucurbit leaf beetle, Colorado potato beetle, rice water weevil, rice weevil, azuki bean weevil, Japanese beetle , soybean beetle, Diabrotica sp., cigarette beetle, powder post beetle, pine sawyer, white-spotted longicorn beetle, Agriotis sp., 28-spotted ladybeetle, rust-red flour beetle, and cotton boll weevil are exemplified. For examples of Dipterous harmful insects, housefly, Calliphora lata, Boettcherisca peregrina, cucurbit fruit fly, citrus fruit fly, seed maggot, rice leaf miner, yellow drosophila, Stomoxys calcitrans, Culex tritaeniarhynchus, Aedes aegypti, and Anopheles hyrcanus, are exemplified. For example of Thysanopterous pest insects, Thrips palmi, and tea thrips are exemplified. For examples of Hymenopterus harmful insects, Monomorium pharaonis, yellow harnet and cabbage sawfly are exemplified. For examples of Orthopterous harmful insects, German cockroach, American cockroach, Japanese cockroach, and grasshopper are exemplified. Isopterous harmful insects, such as Formosan subterranean termite and Reticulitermes speratus Kolbe , aphanipterous harmful insects such as human flea, anoplurous harmful insects such as human louse, mites, such as two-spotted spider mite, carmine mite, Kanzawa spider mite, citrus red mite, European red mite, citrus rust mite, apple rust mite, Tarsonemus sp., Brevipalpus sp., Eotetranychus sp., Robin bulb mite, common grain mite, Desmatophagoides farinae, Boophilus microplus and Haemaphysallis bispinosa, plant-parasitic nematodes, such as southern root-knot nematode, root lesion nematode, soybean cyst namatode, rice white-tip nematode and pine wood nematode are exemplified.

For the pest insects as exemplified above, the compounds of the present invention has all of excellent adulticidal, nymphicidal, larvicidal and ovicidal activities. Recently, a problem has been raised in the control of the pest insects, such as diamond back, planthoppers, leafhoppers, aphids, and phytophagous mites, since these insects and mites have developed the resistance to insecticides, such as organophosphorus insecticides, carbamate insecticides and acaricides, whereby the activity of those insecticides and acaricides have been decreased. Therefore, a chemical which can be effective against such resistant insects and mites has been desired. The compound of the present invention has an excellent insecticidal and acaricidal activities not only against susceptible strains of those insects and mites but also against the resistant strains of those insects and mites to organophosphorus compounds, carbamate compounds, pyrethroid compounds.

The compounds of the present invention can show excellent fungicidal activity against wide varieties of fungi, and therefore, the compounds can be useful for plant disease control in the farming of agricultural and horticultural crops including ornamental flowers, turf and forage crops.

The examples for the plant diseases to be controlled with the compounds of the present invention are given in the following.

| | | |
|---|---|---|
| Paddy rice | Blast | (Pyricularia oryzae) |
| | Sheath blight | (Rhizoctonia solani) |
| | Bakanae disease | (Gibberella fujikuroi) |
| | Helminthosporium leaf spot | (Cochliobolus miyabeanus) |
| Barley | Loose smut | (Ustilago nuda) |
| Wheat | Scab | (Gibberella zeae) |
| | Leaf rust | (Puccinia recondita) |
| | Eye spot | (Pseudocercosporella herpotrichoides) |
| | Glume blotch | (Leptosphaeria nodorum) |
| | Powdery mildew | (Erysiphe graminis f. sp. tritici) |
| | Fusarium snow blight | (Micronectriella nivalis) |
| Potatoes | Late blight | (Phytophthora infestans) |
| Ground nuts | Leaf spot | (Cercospora beticola) |
| Cucumbers | Powdery mildew | (Sphaerotheca fuliginea) |
| | Sclerotinia rot | (Sclerotinia sclerotiorum) |
| | Gray mold | (Botrytis cinerea) |
| | Downy mildew | (Pseudoperonospora cubensis) |
| Tomatoes | Leaf mold | (Cladosporium fulvum) |
| | Late blight | (Phytophthora infestans) |
| Egg plant | Black rot | (Corynespora melongenae) |
| Onions | Gray-mold neck rot | (Botrytis allii) |
| Strawberries | Powdery mildew | (Sphaerotheca humuki) |
| Apples | Powdery mildew | (Podosphaera leucotricha) |
| | Scab | (Venturia inaequalis) |
| | Blossom blight | (Monilinia mali) |
| Oriental persimmon | Anthracnose | (Gloeosporium kaki) |
| Peach | Brown rot | (Monilinia fructicola) |
| Grape | Powdery mildew | (Uncinula necator) |
| | Downy mildew | (Plasmophora viticola) |
| Pears | Rust | (Gymnosporangium asiaticum) |
| | Black spot | (Alternaria kikuchiana) |
| Tea plant | Leaf spot | (Pestalotia theae) |
| | Anthracnose | (Colletotrichum theae-sinensis) |
| Tangerines | Scab | (Elsinoe fawcetti) |
| | Blue mold | (Penicillium italicum) |
| Turf | Sclerotinia snow blight | (Sclerotinia borealis) |

Recently, in the various disease, the resistivities thereof against agents of benzimidazole, agents of carboxyimide and agents of acylalanine have been developed and the efficacy of these agrochemicals become deficient.

It is expected for business to have an agrochemical with satisfactory efficacy against disease belonging to resistive systems. The compounds of this invention possess an excellent activity as a fungicide not only for the fungi sensitive systems but also for resistive systems against the agents of benzimidazole, carboxyimide and acylalanine.

Against the diseases described as follows is a more preferable application of the compounds, such as wheat powdery mildew, apple scab, cucumber mildew and grape mildew and the like.

In addition, these compounds of this invention are useful for an anti-contaminant to prevent the adhering of aquatics on the surfaces of such as vessel-board, fishing nets and the like to add these compounds into shipboard paint.

Another application of these compounds of this invention for a mold protecting agents (moldicide) is also probable and as an example it is preferable to apply the mold protection on the walls of rooms and bath-rooms or in shoes and in clothes by inserting the compounds into paints or fibers.

Further certain compounds of this invention may exhibit activity for an insecticide, acaricide and herbicide.

For practical usage of these compounds obtained in the present invention it is possible to apply with or without another components or vehicles, and also it is preferable to formulate in the mode having cetain formulations performed usually in the field of agrochemicals, preferable modes are such as hydrate, tablet, powder, emulsion, suspension, flowable, and the like, according as the object for use. For the object to apply in solid state it is preferable to formulate with the materials as an additional bulking agents and a carrier. Some examples of materials which can serve for this include: plant origines such as soy bean powder and flour, mineral bases fine powder such as diatomaceus earth, apatite, gypsum, talc, bentonite, pyrofrit, clay and the like, organic and inorganic chemical sources such as sodium benzoate, urea, sodium sulfate and the like. Another examples of materials which can serve as the oject of liquid formulation include: petroleum bases such as kerosene, xylene, solvent naphtha, and the like, and cyclo hexane, cyclo hexanone, dimethylformamide, dimethyl sufoxide, alcohol, acetone, trichloroethylene methyl isobutyl ketone, mineral oil, vegetable oil, water and the like. It may be further possible the addition of some surfactants if it is requested to obtain the more homogeneous and stable mode of agents.

It is further more preferable to keep the effective ingredient of these compounds in the range of 5 to 70 percent. It is further specific object of the present invention that agents such as wettable powder, emulsifiable concentrate and flowable are provided by diluting with water to the selected concentration of a suspension or a emulsion before use, otherwise the tablets and powders are used directly spreading thereof to the plants.

Agrochemical compositions of this invention suitable for plants comprise within these compounds described hereinbefore, however the combination including another one or more of the compounds which are existing in the market may be preferable for various fungicide, insectcide, acaricides and cooperative agent.

Suitable agrochemicals which can be used in combination with the compounds of the present invention such as fugicide, insecticide, acaricide, nematicide and plants growth regulators may be illustrated as follows

### Fungicide:

### Copper agents:

basic copper chloride, basic copper sulfate etc.

### Sulfer agents:

Thiram, Maneb, Mancozeb, Polycarbamate, Propineb, Ziram, Zineb, etc.

### Polyhaloalkylthio agents:

Captan, Dichlorfuranide, Forpet, etc.

### Organic chloride agents:

Chlorotalonyl, Futharide, etc.

### Organophosphrus agents:

IBP, EDDP, Trichlofos-methyl, Pyrazophos, Fosetyl, etc.

### Benzimidazole agents:

Thiophanate-methyl, Benomil, Carbendazim, Thiabendazol, etc.

### Dicarboxyimide agents:

Iprodione, Vinclozolin, Prothimidon, Fluorimide, etc.

### Dicarboxyamide agents:

Oxycarboxin, Mepronyl, Flutoranyl, Tecrofutaram, Trichlamide, Penthichlon, etc.

### Acyalanine agents:

Metharaxyl, Oxadixyl, Flaraxyl, etc.

### SBI agents:

Triazimefon, Triazimenol, Bitertanol, Microbutanil, Hexaconazole, Propiconazole, Triflumizole, Prochloraz, Pefurazoate, Phenalimol, Pryfenox, Triforin, Flusilazole, Ethaconazole, Dichloptrazole, Fluotrimazol, Flutriafen, Penconazole, Diniconazole, Diproconazole, Imazaryl, Tridemorph, Fenpropymorph,
Buthiobate, etc.

### Antibiotics agents :

Polyoxin, Blasticidin S, Kasugamycin, Valinomycin, Sulfric aciddihydrostreptomycin, etc.

### Others :

Propamocarb-HCl salt, Quintozene,Hydroxyisoxazole, Methasulfocarb, Anilazine, Isoprothiorane, Probenazole Quinomethionate, Dithianon, Dinocap, Dichlomezine, Mepanipyrim, Ferimzone, Fluazinam, Pyroquiron, Tricyclazole, Oxolinic acic, Dithianon, Iminoctadine-acetic acid salt, Cymoxanyl, Pyrolnitrin, Methasulfocarb, Diethofencarb, Binapacryl, Lecithin, Sodium bicarbonate, Phenaminosulf, Dozin, Dimethomorph, Phenazinoxyde, etc.

### Insecticide and acaricides:

### Organophosphorus Insecticide and Carbamate Insecticide:

Fenthion, Fenitrothion, Diazinon, Chlorpyrifos, ESP, Vamidothion, Fenthoate, Dimethoate, Formothion, Malathion, Trichlorfon, Thiomethon, Phosmet, Dichlorvos, Acephate, EPBP, Methylparathion, Oxydimetonmethyl, Ethion, Salithion, Cyanophos, Isoxathion, Pyridafenthion, Phosalon, Methidathion, Sulprofos, Chlorfenvinphos, Tetrachlorvinphos, Dimethylvinphos, Propaphos, Isofenphos, Ethylthiomethon, Profenofos, Pyrachlofos, Monoclotophos, Adinphosmethyl, Aldicarb, Methomyl, Thiodicarb, Carbofuran, Carbosulfan, Benfuracarb, Furathiocarb, Propoxur, BPMC, MTMC, MIPC, Carbaryl, Pyrimicarb, Ethiofencarb, Fenoxycarb, Cartap,
Thiocyclam, Bensultap, etc.

### Pyrethroids agents:

Permethrin, Cypermethrin, Deltamethrin, Fenvalerate, Fenpropathrin, Pyrethrin, Allethrin, Tetramethrin, Resmethrin, Dimethrin, Propathrin, Phenothrin, Prothrin, Fulvalinate, Cyfluthrin, Cihalothrin, Flucythrinate, Ethofenprox, Cycloprothrin, Tralomethrin, Cyrafluofen, Profenprox, Acrynathrin,
Fubufenprox, etc.

### Benzoyl urea and other insecticide :

Diflubezuron, Chlorfluazuron, Hexaflumron, Triflumron, Tetrabenzron, Flufenoxuron, Flucyclouron, Buprofezin, Pyriproxyfen, Mehoprene, Endosulfan, Diaferthiuron, Imidacloprid, Fipronyl, Nicotine sulfate, Rotenone, Metaldehyde, Petroleum oil, BT, Microbial insecticide such as insect disease virus, etc.

### Nematicide :

Phenamiphos, Fosthiazate, etc.

### Acaricide :

Chlorbenzylate, Phenisobromolate, Dicofol, Amitraz, BPPS, Benzomate, Hexythiazox, Fenbutatin oxide, Polynactins, Quinomethionate, CPCBS, Tetradifon, Aermectin, Milbemectin, Clofentezin, Cyhexatin, Pyridaben, Fenpyroximate, Tebufenpyrad, Pyrimidifen, Phenothiocarb, Dienochlor, etc.

### Plants growth regulators:

Gibberellins (such as Gibberellin A3, Gibberellin A4, Gibberellin A7), IAA, NAA.

Several examples as an embodiment of the composition of the present invention describe and illustrate the methods for the preparation of the compositions, as well as other aspects of the present invention, and results achieved thereby. Both the components used herein and the ratio thereof in these example are intended to be merely illustrative of the present invention, and not limiting thereof in either scope or spirit. Those of skilled in the art will readily understand the known variation of the composition.

The term "parts" used herein means a weight parts.

### Example 17 Wettable powder

| | |
|---|---|
| The compound of this invention | 40 parts |
| Diatomaceous earth | 53 parts |
| Sulfate ester of higher alcohol | 4 parts |
| Alkylnaphthalenesulfonic acid salt | 3 parts |

After mixing these components homogeneously and grinding finely, the wettable powder having the effective ingredient of 40 % was obtained.

### Example 18 Wettable powder

| | |
|---|---|
| The compound of this invention | 10 parts |
| Diatomaceous earth | 80 parts |
| Sulfate ester of higher alcohol | 5 parts |
| Silica | 5 parts |

After mixing these components homogeneously and grinding finely, the wettable powder having the effective ingredient of 10 % was obtained.

### Example 19 Emulsifiable concentrate

| | |
|---|---|
| The compound of this invention | 30 parts |
| Polyoxyethylene alkyl aryl ether | 7 parts |
| Dimethylformamide | 30 parts |
| Xylene | 33 parts |

After mixing and solubilizing, the emulsifiable concentrate having the effective ingredient of 30 % was obtained.

### Example 20 Emulsifiable concentrate

| | |
|---|---|
| The compound of this invention | 10 parts |
| Alkylphenyl polyoxyethylene | 5 parts |
| Dimethylformamide | 50 parts |
| Xylene | 35 parts |

After mixing and solubilizing, the emulsifiable concentrate having the effective ingredient of 10 % was obtained.

### Example 21 Dusts

| | |
|---|---|
| The compound of this invention | 10 parts |
| Talc | 89 parts |
| Polyoxyethlene alkyl aryl ether | 1 part |

After mixing homogeneously and grinding thereof finely the dusts having the effective ingredient 10 % was obtained.

### Example 22 Dusts

| | |
|---|---|
| The compound of this invention | 5 parts |
| Talc | 94.7 parts |
| Silica | 0.3 parts |

After mixing homogeneously and grinding thereof finely, the dusts having the effective ingredient 5 % was obtained.

### Example 23 Granules

| | |
|---|---|
| The compound of this invention | 5 parts |
| Clay | 73 parts |
| Bentonite | 20 parts |
| Sodium dioctylsulfosuccinate | 1 part |
| Sodium phosphate | 1 part |

After mixing and grinding thereof, water was added into said mixture and was kneaded homogeneously. The granules having the effective ingredient of 5 % was obtained after granulation and drying.

### Example 24 Flowable concentrate

| | |
|---|---|
| The compound of this invention | 10 parts |
| Ligninsulfonic acid sodium salt | 4 parts |
| Sodium dodecylbenzensulfonate | 1 part |
| Xanthanegum | 0.2 parts |
| Water | 84.8 parts |

After mixing, the mixture was grinded with water until the size of said particles became less than one micron and the flowable concentrate with 10 % effective ingredient was obtained.

### Industrial Applicabity:

### Test 1. Efficacy toPseudaletia separata Walker

According to the formulation described in Example 17, wettable powder was diluted with water until the concentration of the compound of this invention became 125 ppm.

Into said chemical solution corn leaves were steeped for 30 seconds, and were dried under atomosphere. Said leaves then were placed into the petridish wherein 5 second instarlarvae of Pseudaletia separata Walker. The petridish was covered and was kept for 5 days in a thermostatic room with temperature of 25°C and relative humidity of 65 %. After 5 days the lethal rate was examined. The test was a repeating double trial. The compound illustrated hereinafter were displayed more than 80 % of the lethal rate.

Compound No. : I - 2, I - 3, I - 4, I - 5, I - 6, I - 9, I - 15, I - 20, I - 37, I - 50, I - 53, I - 62, I - 71, I - 76, I - 87, I - 89, I - 90, I - 94, I - 99, I - 102, I - 106, I - 107, I - 109, I - 113, I - 114, I - 116, I - 117
II - 1, II - 8, II - 15, II - 27, II - 67, II - 80, II - 81, II - 108, II - 130, II - 132, II - 149, II - 156, II - 160, II - 162, II - 165, II - 166, II - 168, II - 169

### Test 2. The efficacy to Plutella xylostella linné

According with the formulation of wettable powder described in example 17, the wettable powder was diluted with water until the concentration of the compounds in this invention became 125 ppm. Into these chemical solutions the leaves of cabbage were steeped for 30 seconds and were dried under atomosphere. The leaves were placed into the petridish wherein 10 second instarlarvae of Plutella xylastella linné. The petridish was covered and was kept in a thermostatic room with the condition of 25 °C and 65 % relative humidity. After 5 days the lethal rate of the worms were examined. The results of test made by a repeating double trial method were illustrated as follows.

Compound No.: I - 1, I - 2, I - 3, I - 4, I - 5, I - 15, I - 20, I - 37, I - 62, I - 71, I - 76,
II - 1, II - 3, II - 4, II - 5, II - 6, II - 10, II -15, II - 27, II - 67, II - 80, II - 108

### Test 3. The efficacy to Aphis gossypii Glover

The seedlings of cucumber after 10 days from seeding in 3 inches pots were inoculated by Aphis gossypii Glover.

After 1 day the adults were removed. The cucumber seedlings thereto born nymphs were parasitic, were sprayed by the chemical solutions which were prepared according to the formulation of emulsifiable concentrate described in example 19 and were diluted with water to the concentration of 125 ppm. Then the seedlings of cucumber was placed in a thermostatic room with the condition of 25 °C and 65 % relative humidity. After 6 days of duration, the lethal rate were examined. The test was carried out by a repeating double trial and the compounds which exhibit more than 80 % lethal rate were illustrated as follows.

Compound No.: I - 3, I - 4, I - 5, I - 6, I - 8, I - 9, I - 20, I - 22, I - 27, I - 29, I - 32, I - 37, I - 62, I - 87, I - 94, I - 96, I - 102, I - 107, I - 108, I - 109, I - 113, I - 114
II - 6, II - 30, II - 47, II - 50, II - 55, II - 56, II - 108, II - 149, II - 162

### Test 4. The efficacy to Nilaparvata lugens Stal

According with the formulation described in Example 19 of emulsifiable concentrate, the chemical solutions were prepared by dilution with water to the concentration thereof about 125 ppm. Into said solution the seedlings of rice which was 7 days after germination were steeped for 30 seconds and were dried under atmosphere. These treated seedlings were placed into a test tube and 10 fourth instar hymphs Nilaparvata lugens Stal were also inoculated on rice leaves. The test tubes were covered with gauze. Then test tubes were placed in a thermostatic room with the condition of 25°C and 65 % relative humidity. After 5 days duration the lethal rate were examined. These experiments were proceeded by a repeating double trial and the effective compound which achieved more than 80 % of lethal rate were illustrated as follows.

Compound No.: I - 1, I - 3, I - 5, I - 6, I - 7, I - 8, I - 9, I - 15, I - 25, I - 26, I - 28, I - 29, I - 39, I - 43, I - 45, I - 50, I - 52, I - 63, I - 64, I - 76, I - 87, I - 89, I - 96, I - 102, I - 107, I - 109, I - 113, I - 114, I - 115, I - 117, I - 118
II - 4, II - 23, II - 29, II - 47, II - 55, II - 56, II - 67, II - 77, II - 80, II - 81, II - 105, II - 108, II - 129, II - 149, II - 156, II - 164, II - 165, II - 166, II - 167, II - 169, II - 170

### Test 5. The efficacy to Tetranychus uriticae Koch

On the primary leaf of the seedling of Kidney beans which beeing seeded on 3 inches pots were in 7 to 10 days growth after germination. 17 adult of Tetranychus uriticae Koch having the to the organophosphorus agrochemicals were inoculated. According to the formulation of wettable powder described in Example 17, the wettable powder was diluted with water until said concentration reached to 125 ppm and was distributed on seedlings. Then these plants were placed in a thermostatic room with the condition of 25°C and 65 % relative humidity.

After 3 days duration the lethal rate were examined and the test was a repeating double trial. The result obtained and the compounds which achieved more than 80 % of lethal rate were illustarated as follows.

Compound No.: I - 1, I - 2, I - 3, I - 5, I - 6, I - 9, I - 15, I - 25, I - 26, I - 32, I - 33, I - 37, I - 50, I - 52, I - 55, I - 56, I - 57, I - 62, I - 63, I - 64, I - 71, I - 76, I - 87, I - 91, I - 92, I - 94, I - 96, I - 102, I - 106, I - 107, I - 108, I - 109, I - 113, I - 114, I - 116, I - 118
II - 1, II - 6, II - 27, II - 29, II - 30, II - 47, II - 50, II - 56, II - 61, II - 64, II - 66, II - 80, II - 81, II - 82, II - 105, II - 108, II - 129, II - 139, II - 140, II - 170

### Test 6 The trial protection for the scab disease of apple

The young plants of apple cultivated in ceramic pots belonging to species "kokko" which were in the third to fourth leaf stage, were distributed by emulsion agents of the compound in this invention with the concentration of 200 ppm.

After distribution the plants were dried under atmosphere and were transplanted by the derived spores of apple scab disease Venturia inaequalis and were kept in the thermostatic room having a device which was repeating light and dark with a interval of 12 hours alternatively, at the temperature of 20°C for two weeks.

The development of disease spots on the leaves were observed comparing with the untreated leaves and the following compounds demonstrate the distiguished protection activity of more than 80 %.

Compound No.: I - 94, I - 99, I - 106
II - 6, II - 27, II - 30, II - 42, II - 43, II - 47, II - 49, II - 53, II - 66, II - 75, II - 80, II - 108, II - 131, II - 141, II - 149, II - 156

### Test 7 The trial protection for the powdery mildew desease of wheat

The young plants of wheat cultivated in ceramic pots belonging to species "Chihoku" which were in the 1.0 to 1.2 leaf stage, were distributed by emulsion agents of the compound in this invention with the concentration of 200 ppm.

After distribution the plants were dried under atmosphere, and were transplanted by the derived spores of wheat powdery mildew disease Erysiphe graminis f.sp. tritici, scattering thereof over the wheat. Then the test was conducted at the temperature in range of 22 to 25°C for 7 days.

The generation of disease spots on the leaves were measured in comparison with untreated plants and concluding activity, the following compound achieved the excellent results which proved more than 80 %.

Compound No.: I - 3, I - 5, I - 6, I - 12, I - 14, I - 15, I - 16, I - 18, I - 20, I - 25, I - 27, I - 31, I - 32, I - 33, I - 35, I - 41, I - 42, I - 43, I - 46, I - 47, I - 48, I - 52, I - 56, I - 57, I - 66, I - 71, I - 76, I - 77, I - 80, I - 82, I - 83, I - 84, I - 87, I - 92, I - 94, I - 96, I - 100, I - 102, I - 106, I - 108, I - 109, I - 112
II - 4, II - 8, II - 10, II - 13, II - 15, II - 27, II - 30, II - 35, II - 43, II - 45, II - 46, II - 47, II - 66, II - 80, II - 82, II - 84, II - 86, II - 93, II - 97, II - 108, II - 141, II - 142, II - 143, II - 149, II - 164

### Test 8 The trial protection for the downy mildew disease of cucumber

The young plants of cucumber cultivated in ceramic pots belonging to species "Sagamihanjiro" were distributed by emulsion agents of the compound in this invention with the concentration of 200 ppm. After distribution the plants were dried under atomosphere, said cucumbers were transplanted by the disease of cucumber downy mildew (Pseudoperonospora cubensis) spraying the suspension of said spore. Then the plants were kept in the thermostatic room having a device which was repeating light and dark with a interval of 12 hours alternatively, at the temperature of 25°C for 4 days.

Observing the desease spots generated on the leave between treated and untreated, the activity for protection was concluded and the following compounds illustrated the activity of more than 80 %.

Compound No.: I - 8, I - 57
II - 3, II - 45, II - 57, II - 59, II - 85, II - 97, II - 142, II - 143

### Test 9 The trial protection for the downy mildew disease of grape

Punching holes with a diameter of 30 mm on the leaves of usual plants of grapes cultivated without any housings which were belonged to species "Kaiji" and in three years growth, the removed leaves were used for trial dipping them into the solutin therein the conpound of this invention as an emulsion was diluted with concentration of 200 ppm.

The wet leaves were dried at room temperature naturally and were inoculated by spraying the migrating spore of the disease in grape downy mildew Plasmopara viticola and stored in a thermostatic room under lighting at temperature of 20°C for 10 days.

After the measureament for the growth of disease spots on the leaves between treated and untreated, compounds which achieved more than 80 % of activity were illustrated as follows.

Compound No.: I - 6, I - 8, I - 9, I - 20, I - 24, I - 59, I - 94
II - 3, II - 36, II - 37, II - 50, II - 83, II - 141, II - 156, II - 164

## Claims

1. An imidazole derivative having the formula [I] 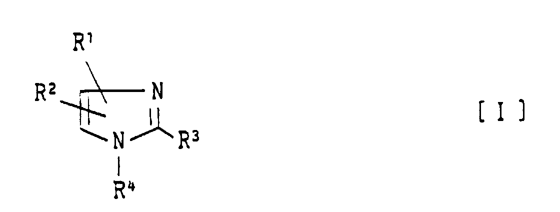 ( wherein R¹ represents phenyl which is optionally substituted with halogen, C₁₋₆ alkyl, C₁₋₆ haloalkyl, C₁₋₆ alkoxy, C₁₋₆ haloalkoxy, C₁₋₆ alkoxy C₁₋₆ alkoxy, optionally substituted phenyl, optionally substituted pyridyl, optionally substituted phenoxy, optionally substituted pyridyloxy, C₂₋₆ alkenyloxy, C₂₋₆ haloalkenyloxy, C₂₋₆ alkynyloxy, C₂₋₆ haloalkynyloxy, nitro, C₁₋₆ alkylthio, C₁₋₆ alkylsulsulfinyl, C₁₋₆ alkylsulfonyl, di-C1-6 alkylsulfamoyl, cyano, C₁₋ ₆ alkoxycarbonyl or C₁₋₆ alkylcarbonyl or this pheny group is condensed with benzene to represents naphthyl optionally; and
R² represents C₁₋₆ alkoxycarbonyl C₁₋₆ alkoxy C₁₋₆ alkoxycarbonyl, cyano; r¹r² NCO group wherein r¹ and r² are each independently hydrogen, C₁₋₆ alkyl or optionally substituted phenyl; C₁₋₆ alkylthiocarbonyl; C₁₋₆ alkoxycarboimidoyl; carboxy; a halogen; C₁₋₆ haloalkyl; formyl or nitro; and
R³ represents phenyl optionally substituted with a halogen; C₁₋₆ haloalkyl; C₁₋₁₂ alkyl; benzoyl; C₁₋₆ alkoxycarbonyl; C₁₋₆ alkylcarbonyl;aralkyl or C₃₋₇ cycloalkyl or hydrogen; and
R⁴ represents hydrogen; aralkyl C₂₋₆ alkenyl; C₂₋₆ alkynyl; C₁₋₆ alkylcarbonyl; cyano or C₁₋₆ alkyl which is optionally substituted with the group selected from C₁₋₆ alkoxy, optionally substituted aralkyloxy, C₁₋₆ alkoxy C₁₋₆ alkoxy, C₁₋₆ acyloxy, C₁₋₆ alkoxycarbonyloxy, C₁₋₆ acylamido, C₂₋₆ alkenyloxy, C₂₋₆ alkynyloxy, r³ SOn (r³ is C₁₋₆ alkyl or optionally substituted phenyl and n is integer of 0, 1, 2), optionally substituted benzoyl, C₁₋₆ alkoxycarbonyl, di-C₁₋₆ alkylphosphinyl. cyano or thiocyanate; or R² and R⁴ are allowed to form a ring.

2. A method for preparing the imidazole derivative having the formula [I-1] 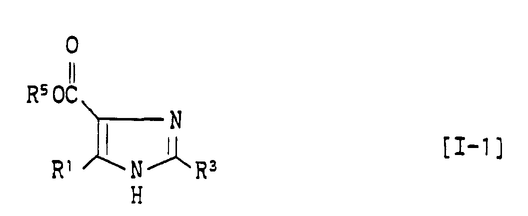 (wherein R¹, R³ and R⁵ are the same meaning as described above)
characterised by the reaction of 3-oxazolidine-5-one derivatives having the formula [II] 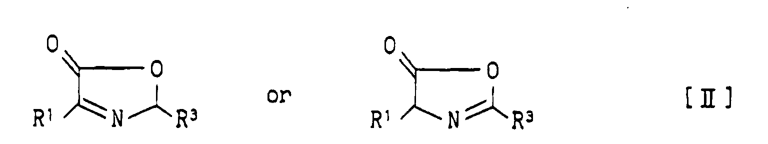 (wherein R¹, R³ represent the same meaning as described above) with alkyl cyanoformate having the formula [II 1]
NCCOOR⁵ [III]
(wherein R⁵ is C₁₋₆ alkyl)
catalyzed by trivalent phosphorous compounds.

3. A method for preparing the imidazole derivative having the formula [I-1] 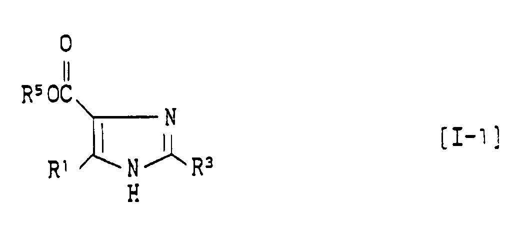 (wherein R¹, R³, R⁵ are the same meaning as described above)
characterised by the reaction of glycine derivatives having the formula [IV] 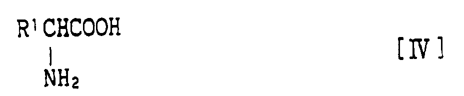 (wherein R¹ represent the same meaning as described above)
with acid anhydride derivative having the formula [V]
(R³CO)₂O [V]
(wherein R³ is the same meaning as described above)
and alkyl cyanoformate having the formula [II 1]
NCCOOR⁵ [III]
(wherein R⁵ is the same as in claim 2)
in the presence of trivalent phosphorous compounds and dehydrogenating agents.

4. A method for preparing the imidazole derivative having the form ula [I-1] 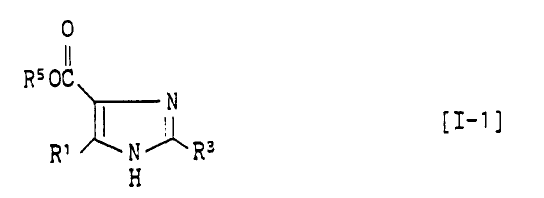 (wherein R¹, R³, R⁵ are the same meaning as described above)
characterised by the reaction of N-acyglycine derivative having the formula [VI] 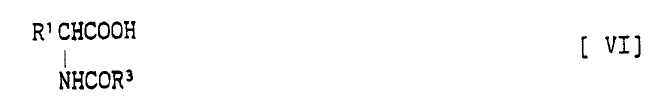 (wherein R¹, R³ represent the same meaning as described above)
with alkyl cyanoformate having the formula [II 1]
NCCOOR⁵ [III]
(wherein R⁵ is the same as in claim 2)
in the presence of trivalent phosphorous compounds and dehydrating agents.

5. A method for preparing the imidazole derivative having the formula [I-2] 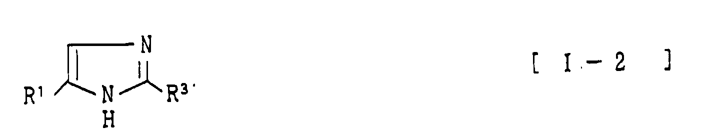 (wherein R¹ and R³ are the same meaning as described above)
characterized by the reaction of amidine derivative having the formula [VII'] 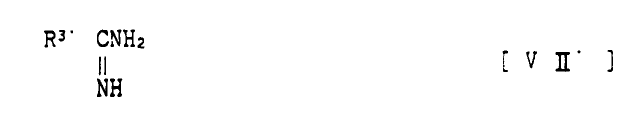 (wherein R³ is C₁₋₆ haloalkyl)
with haloketone having the formula [VIII] 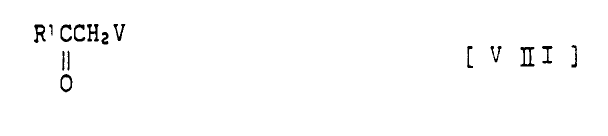 (wherein R¹ is the same meaning as described above and V is halogen.)

6. A composition for insecticide or acaricide characterized by containing one more than two imidazole derivative having the formula [I] as an effective ingredient: 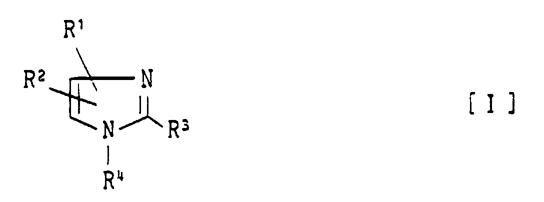 (wherein R¹, R², R³ and R⁴ are the same meaning as described above)

7. A composition of fungicide for use in agriculture and horticulture characterized by containing one or more than two imidazole derivative having the formula [I] as an effective ingredient: 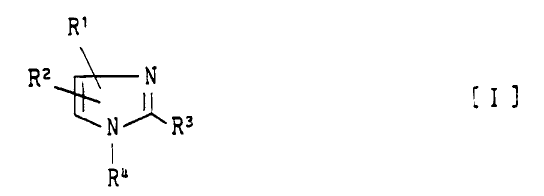 (wherein R¹, R², R³ and R⁴ are the same meaning as described above)
